# EUROPÄISCHE PATENTSCHRIFT

(11) **EP 3 423 515 B1**
(45) Veröffentlichungstag und Bekanntmachung des Hinweises auf die Patenterteilung: **26.08.2020**
(21) Anmeldenummer: 17717060.2
(22) Anmeldetag: 02.03.2017
(51) Int. Cl.: C08J 5/02, C08J 3/26, B29C 41/14, A61B 42/10

(54) **VERFAHREN ZUM HERSTELLEN EINES PROPHYLAXEARTIKELS**
METHOD FOR PRODUCING A PROPHYLACTIC ARTICLE
PROCÉDÉ DE FABRICATION D'UN ARTICLE DE PROPHYLAXIE

(30) Priorität: 04.03.2016 AT 501762016
(43) Veröffentlichungstag der Anmeldung: 09.01.2019
(60) Teilanmeldung: 19210944.5
(73) Patentinhaber: Semperit Aktiengesellschaft Holding, 1031 Wien (AT)
(72) Erfinder: HOLZNER, Armin, 2630 Ternitz (AT); KERN, Wolfgang, 8055 Seiersberg (AT); MANHART, Jakob Cornelius, 2372 Giesshübl (AT); SAHIN, Melahat, 8700 Leoben (AT); SCHALLER, Raimund, 2620 Neunkirchen (AT); SCHLÖGL, Sandra, 8152 Stallhofen (AT)
(74) Vertreter: Burger, Hannes
(86) Internationale Anmeldenummer: PCT/AT2017/060053
(87) Internationale Veröffentlichungsnummer: WO 2017/147639

(56) Entgegenhaltungen:
- EP-A1- 2 719 720
- EP-A2- 1 762 586
- WO-A1-2010/105283
- DIETMAR LENKO ET AL: "Dual crosslinking of carboxylated nitrile butadiene rubber latex employing the thiol-ene photoreaction", JOURNAL OF APPLIED POLYMER SCIENCE, Bd. 129, Nr. 5, 5. September 2013 (2013-09-05), Seiten 2735-2743, XP055387216, US ISSN: 0021-8995, DOI: 10.1002/app.38983
- SANDRA SCHLÖGL ET AL: "Photo-vulcanization using thiol-ene chemistry: Film formation, morphology and network characteristics of UV crosslinked rubber latices", POLYMER., Bd. 55, Nr. 22, 10. Juni 2014 (2014-06-10) , Seiten 5584-5595, XP055387227, GB ISSN: 0032-3861, DOI: 10.1016/j.polymer.2014.06.007

## Beschreibung

Die Erfindung betrifft ein Verfahren zum Herstellen eines Prophylaxeartikels, insbesondere eines Handschuhs, aus einem (carboxylierten) Dienkautschuk, nach dem auf eine Form zumindest eine Schicht aus einem (carboxylierten) Dienlatex aufgebracht wird, und der (carboxylierte) Dienlatex mit einem Vernetzungsmittel vernetzt wird.

Weiter betrifft die Erfindung einen Prophylaxeartikel, insbesondere Handschuh, umfassend eine Schicht aus einem (carboxylierten) Dienelastomer, wobei die (carboxylierten) Dienelastomermolekülketten des (carboxylierten) Dienelastomers kovalent über zumindest ein Polymer vernetzt sind.

Prophylaxeartikel, wie insbesondere Operations- und Untersuchungshandschuhe, werden üblicherweise aus einem Elastomerlatex durch eintauchen von handförmigen Tauchformen hergestellt. Auf den Tauchformen bildet sich ein Film, aus dem in weiterer Folge der fertige Einweghandschuh durch Vulkanisation bzw. Vernetzung des Latex entsteht.

Prophylaxeartikel aus Naturlatex weisen ein relativ hohes Allergiepotential auf. Aus diesem Grund werden vermehrt Syntheselatices zur Herstellung der Prophylaxeartikel verwendet. Aber auch diese sind nicht gänzlich hypoallergen, da sie nach wie vor Allergene aus dem Herstellungsprozess aufweisen können, wie beispielsweise Puder zur Verbesserung der Anziehbarkeit, oder Prozesschemikalien, wie beispielsweise Vernetzungschemikalien oder Vernetzungsbeschleuniger.

Um diesen Problemen zu begegnen, wurden im Stand der Technik bereits Verfahren zur Herstellung von Prophylaxeartikel mit reduziertem Allergiepotential vorgeschlagen.

Beispielsweise beschreibt die WO 2011/068394 A1 ein Verfahren, nach dem einem carboxylierten Nitrilbutadien eine Methacrylsäure und ZnO zugesetzt werden. Dadurch erlangt diese Mischung selbstvemetzende Eigenschaften, sodass auf Schwefelvernetzer und Beschleuniger verzichtet werden kann. Nach wie vor enthält diese Zusammensetzung aber das Schwermetall Zn, sodass ein gewisses Allergierestpotential verbleibt.

Ähnlich dazu beschreibt die US 2010/0152365 A1 die Verwendung eines carboxylierten Nitrilbutadien Copolymers zur Herstellung eines Handschuhs mittels Tauchverfahren. Wiederum wird ZnO zur ionischen Vernetzung eingesetzt.

Es ist weiter bekannt, die Oberfläche von Naturkautschukhandschuhen zu modifizieren, um dessen Allergiepotential zu reduzieren. So beschreibt beispielsweise die von der Anmelderin stammende US 2014/0096307 A1 ein Verfahren zur Modifizierung der Oberfläche eines Elastomers mit ungesättigten Kohlenstoff-Kohlenstoff-Bindungen, die im Bereich der Oberfläche zumindest teilweise durch eine photochemische Reaktion mit zumindest einem Thiol gesättigt werden. Zur Sättigung können Feststoffpartikel verwendet werden, die kovalent an die Oberfläche des Handschuhs gebunden werden.

Ähnlich dazu beschreibt die ebenfalls auf die Anmelderin zurückgehende US 2014/0096308 A1 u.a. die Anbindung von Zeolithpartikel an einen Naturkautschukhandschuh über Epoxidgruppen.

Aus DIETMAR LENKO ET AL: "Dual crosslinking of carboxylated nitrile butadiene rubber latex employing the thiol-ene photoreaction", JOURNAL OF APPLIED POLYMER SCI-ENCE, Bd. 129, Nr. 5, 5. September 2013 (2013-09-05), Seiten 2735-2743, XP055387216, US ISSN: 0021-8995, D01: 10.1002/app.38983 ist ein Verfahren gemäß dem Anspruch 1 bekannt, wobei als Vernetzungsmittel ein mehrfach funktionelles Monomer ("Trimethylolpropantris-3-mercaptopropionate (Tri-Thiol), verwendet wird.

Auch aus SANDRA SCHLÖGL ET AL: "Photo-vulcanization using thiol-ene chemistry: Film formation, morphology and network characteristics of UV crosslinked rubber latices", POLYMER., Bd. 55, Nr. 22, 10. Juni 2014 (2014-06-10), Seiten 5584-5595, XP055387227, GB ISSN: 0032-3861, D01: 10.1016/j.polymer.2014.06.007 ist die Vernetzung eines Latex über die Thiol-En Reaktion mit Trimethylolpropane tris(3-mercaptopropionate) (Tri-Thiol) als Vernetzungsmittel bekannt.

Die EP 1 762 586 A2 beschreibt ein Verfahren zur Herstellung eines vernetzten Elastomers, nach dem eine Mischung umfassend zumindest einen Latex und zumindest eine Starterkomponente zur Auslösung der Vernetzungsreaktion oder ein Latex der zumindest eine Starterkomponente bzw. Startergruppe trägt hergestellt wird, insbesondere in flüssiger Phase, wobei als Starterkomponente ein Fotoinitiator eingesetzt wird, der durch Bestrahlung mit elektromagnetischer Strahlung im ultravioletten (UV-Licht) und/oder sichtbaren Spektralbereich eine reaktive Spezies bildet, und die Latexmischung mit UV-Licht und/oder sichtbaren Licht bestrahlt wird. Als Vernetzungshilfsmittel kann zumindest ein Thiol und/oder ein Selenol, insbesondere jeweils mehrfach funktionelle Derivate davon, sowie Mischungen daraus, zugesetzt werden. Das Thiol kann ausgewählt sein aus einer Gruppe umfassend Trimethylolpropan-tris-(3-mercaptopropionat), 1,6-Hexandithiol, sowie Mischungen daraus. Weiter kann ein weiteres Vernetzungshilfsmittel zugesetzt sein, ausgewählt aus einer Gruppe umfassend, insbesondere mehrfach funktionelle, Acrylate, wie z.B. Hexandioldiacrylat, Trimethylolpropantriacrylat, Verbindungen mit Vinyl- oder Allylgruppen, wie z.B. Triallyl-Cyanurat, Triallyl-Isocyanurat, sowie Mischungen daraus.

Die WO 2010/105283 A1 beschreibt ein Verfahren zur Herstellung eines vernetzten Elastomers durch Bestrahlung einer Polymerdispersion aus zumindest einem vernetzbaren Polymer mit elektromagnetischer Strahlung im ultravioletten (UV-Licht) und/oder sichtbaren Spektralbereich, wobei die Vernetzung in zumindest zwei Schritten als Vorvernetzung und Nachvernetzung durchgeführt wird und der Polymerdispersion zumindest ein Fotoinitiator zur Auslösung der Vernetzungsreaktion vor der Vorvernetzung zugesetzt wird, wobei weiter der vorvernetzten Polymerdispersion vor und/oder während der Nachvernetzung nochmals zumindest ein Fotoinitiator zugesetzt und die Nachvernetzung ebenfalls mit elektromagnetischer Strahlung im ultravioletten (UV-Licht) und/oder sichtbaren Spektralbereich durchgeführt wird. Der Vorvernetzung und/oder der Nachvernetzung kann zumindest ein Covemetzer mit zumindest einer Thiolgruppe zugesetzt werden. Als Covemetzer kann Tri-methylolpropan-tris-3-mercaptopropionat oder Pentaerythritol tetrakis-3- mercaptopropionat eingesetzt werden.

Der vorliegenden Erfindung liegt die Aufgabe zugrunde, einen verbesserten Prophylaxeartikel zu schaffen.

Die Aufgabe wird bei dem eingangs genannten Verfahren dadurch gelöst, dass als Vernetzungsmittel ein mercaptofunktionelles Siloxanpolymer verwendet wird.

Weiter wird die Aufgabe durch den eingangs genannten Prophylaxeartikel gelöst, bei dem das zumindest eine Polymer ein mercaptofunktionelles Siloxanpolymer ist.

Von Vorteil ist dabei, dass durch die chemische Reaktion des Vernetzungsmittels mit den Elastomermolekülen das Vernetzungsmittel besser in das vernetzte Elastomer eingebaut wird.

Dadurch ist das Vernetzungsmittel nicht bzw. schwer aus dem Elastomer extrahierbar bzw. migriert nicht und nur sehr langsam aus dem Elastomer. Mit "sehr langsam" ist dabei gemeint, dass die Migrationszeit sehr viel größer ist, als die Anwendungszeit des Prophylaxeartikels. Es wird damit verhindert, dass das Vernetzungsmittel in Kontakt mit der menschlichen Haut gelangt, wodurch das Allergiepotential des Prophylaxeartikels deutlich gesenkt werden kann. Auch während der Lagerung der Prophylaxeartikel kann die Migration des Vernetzungsmittels aus dem Prophylaxeartikel verhindert bzw. deutlich verringert werden. Zudem können dadurch Leachingprozesse zur Entfernung nicht gebundener Prozesschemikalien verkürzt oder sogar eingespart werden. Mit dem Verfahren kann ein Prophylaxeartikel hergestellt werden, der sehr gute mechanische Eigenschaften und eine hohe Alterungs- und Gammabeständigkeit aufweist. Auch konnte eine Beeinflussung der Filmbildung während des Herstellungsprozesses, insbesondere des Tauchprozesses, nicht nachgewiesen werden, sodass also keine weiteren Maßnahmen hierfür erforderlich sind. Ein weiterer Vorteil des Verfahrens ist darin zu sehen, dass keine Vorvernetzung des (carboxylierten) Dienlatex erforderlich ist, sodass kontinuierliche Mischverfahren eingesetzt und die Prozessabläufe beschleunigt werden können. Es ist mit dem Verfahren eine energieeffiziente, nachhaltige und produktionseffiziente Herstellung von hypoallergenen Prophylaxeartikeln, insbesondere Operations- und Untersuchungshandschuhen, möglich. Durch die Wasserlöslichkeit des Vernetzungsmittels wird bei dessen Einbringung in die Latexmischung kein bzw. nicht zwingend ein Emulgator benötigt. Zudem kann auch der Modul des Prophylaxeartikels besser eingestellt werden.

Die Vernetzung der (carboxylierten) Dienlatexmoleküle kann thermisch durchgeführt werden. Somit kann die Vernetzung der Latexmoleküle bereits während des Trocknens des auf die Tauchform aufgetauchten Latexfilms erfolgen, wodurch eine Effizienzsteigerung des Verfahrens erzielbar ist.

Es ist weiter möglich, die Vernetzung der (carboxylierten) Dienlatexmoleküle photochemisch mittels UV-Licht durchzuführen. Es kann damit die Alterungsbeständigkeit des Elastomers verbessert werden. Auch hinsichtlich hochenergetischer Strahlung weisen die Elastomerprodukte eine verbesserte Stabilität auf. Dies ist insbesondere in Hinblick auf die Sterilisation der Medizinprodukte mit Gammastrahlung von Bedeutung. Zudem kann durch dieses Verfahren der Einsatz von Typ IV allergenen Substanzen ebenfalls einfacher vermieden werden.

Vorzugsweise wird der pH-Wert des (carboxylierten) Dienlatex auf einen Wert von größer/gleich 9 eingestellt. Es konnte mit pH-Werten ab 9 eine deutliche Verbesserung der Reaktionskinetik beobachtet werden, wodurch die Vernetzung der Moleküle rascher erfolgen kann.

Es besteht auch die Möglichkeit, das Vernetzungsmittel dem (carboxylierten) Dienlatex als Emulsion zuzusetzen. Durch die feine Verteilung des Vernetzungsmittels in der Emulsion kann ein homogenerer Prophylaxeartikel einfacher erreicht werden.

Als mercaptofunktionelles Siloxanpolymer kann gemäß einer Ausführungsvariante ein mercaptofunktionelles Siloxanhomopolymer oder ein Copolymer des mercaptofunktionellen Siloxanhomopolymers mit einem Acrylsiloxan verwendet werden. Im Zuge von durchgeführten Tests haben sich diese Polymere bereits bei geringen Konzentrationen als positiv für die mechanischen Eigenschaften des damit vernetzten Prophylaxeartikels herausgestellt.

Besonders bevorzugt wird gemäß einer Ausführungsvariante des Verfahrens ein mercaptofunktionelles Siloxanhomopolymer mit der Strukturformel 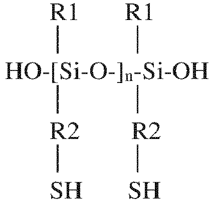 eingesetzt, wobei R1 für eine erste Einheit ausgewählt aus einer ersten Gruppe bestehend aus -CH₃, -OH, -C₂H₅, -C₃H₇, aromatische Gruppen, R2 für eine zweite Einheit ausgewählt aus einer zweiten Gruppe bestehend aus -CH₂, C₂H₄, C₃H₆; -(CH₂)₁₁-, aromatische Gruppen, - CH₂-Aromat, steht. Ebenso wird besonders bevorzugt gemäß einer weiteren Ausführungsvariante des Verfahrens ein mercaptofunktionelles Siloxancopolymer, insbesondere ein mercaptofunktionelles Siloxancopolymer mit einer statistischen Anordnung der Wiederholungseinheiten, mit der Strukturformel 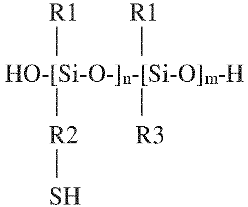 eingesetzt, wobei R1 für eine erste Einheit ausgewählt aus einer ersten Gruppe bestehend aus -CH₃, -OH, -C₂H₅, -C₃H₇, aromatische Gruppen, R2 für eine zweite Einheit ausgewählt aus einer zweiten Gruppe bestehend aus -CH₂, C₂H₄, C₃H₆; -(CH₂)₁₁-, aromatische Gruppen, - CH₂-Aromat, und R3 für eine dritte Einheit ausgewählt aus einer dritten Gruppe bestehend aus Alkylgruppen (-CH₃, -C₂H₅, --C₃H₇, etc.), -CH₂-Aromat, aromatische Gruppen, Alkengruppen (-CH=CH₂, -CH₂CH=CH₂, etc), Methacryloxypropyl-, Acryloxypropyl-, Epoxygruppen (Epoxycyclohexylethyl, Glycidoxypropyl) steht. Durch diese Polymere können die voranstehend genannten Effekte weiter verbessert werden.

Als mercaptofunktionelles Siloxan kann auch das nachstehende Dimer eingesetzt werden.

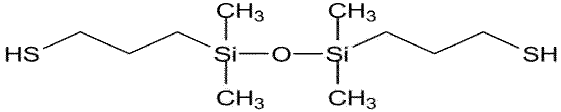

Im Zuge der Arbeiten im Zusammenhang mit dieser Erfindung wurde gefunden, dass es von Vorteil ist, wenn das mercaptofunktionelle Siloxanhomopolymer ausgewählt ist aus einer Gruppe bestehend aus Poly(mercaptopropyl)siloxan, Poly(mercaptomethylpropyl)siloxan, Poly(mercaptomethylmethyl)siloxan, Poly(mercaptoethylmethyl)siloxan, Poly(mercaptomethylethyl)siloxan, Poly(mercaptopropylmethyl)siloxan, Po-ly(mercaptomethylbenzyl)siloxan, Poly(mercaptopropylbenzyl)siloxan, Po-ly(mercaptoethylbenzyl)siloxan und/oder das Copolymer des mercaptofunktionellen Siloxanhomopolymers mit einem Acrylsiloxan ausgewählt ist aus einer Gruppe bestehend aus Po-ly(mercaptomethylpropyl-co-acryloxymethylpropyl)siloxan, Poly(mercaptomethylmethyl-co-acryloxymethylpropyl)siloxan, Poly(mercaptomethylmethyl-co-acryloxypropylmethyl)siloxan, Poly(mercaptomethylmethyl-co-acryloxypropylethyl)siloxan, Poly(mercaptomethylmethyl-co-acryloxyethylpropyl)siloxan, Poly(mercaptomethylmethyl-co-acryloxymethylmethyl)siloxan, Poly(mercaptomethylmethyl-co-acryloxypropyl)siloxan, Poly(mercaptomethylmethyl-co-acryloxyethyl)siloxan, Poly(mercaptomethylmethyl-co-acryloxymethyl)siloxan, Poly(mercaptopropylmethyl-co-acryloxymethylpropyl)siloxan. Von Vorteil ist dabei, dass diese Verbindungen eine hohe Mercaptoequivalentzahl und damit eine hohe Reaktivität bei der Vernetzung aufweisen. Zudem sind sie flüssig und können damit einfach in das System über Emulsion eingebracht werden. Darüber hinaus sind sie geruchslos und beeinflussen auch in hoher Konzentration die Filmbildung nicht. Durch den polaren Charakter der SH-Gruppen sind die Emulsionen auch über lange Zeit stabil.

Durch die Acrylatgruppen wird einerseits verhindert, dass es während der UV-Vernetzung vermehrt zur Disulfidbildung (über benachbarte Einheiten) kommt und andererseits kann die Acrylatgruppe ebenfalls mit dem Kautschuk im Zuge der Thiol-En Reaktion reagieren.

Nach einer weiteren Ausführungsvariante des Verfahrens kann vorgesehen sein, dass der Anteil des mercaptofunktionellen Siloxanpolymers an dem Copolymer des mercaptofunktionellen Siloxanpolymers mit einem Acrylsiloxan zumindest 20 Gew.-% beträgt. Es können damit die voranstehend genannten Vorteile/Effekte besser erreicht werden.

Das Vernetzungsmittel kann dem (carboxylierte) Dienlatex in einem Anteil von 1 phr bis 10 phr, bezogen auf die Gesamtzusammensetzung des (carboxylierten) Dienlatex, zugesetzt werden. Es kann damit besser vermieden werden, dass der 50 % Modul zu hoch wird, wodurch die Tragbarkeit des Prophylaxeartikels leiden würde. Gleichzeitig werden aber mit einer Konzentration des Vernetzungsmittels aus diesem Bereich gute mechanische weitere Eigenschaften des Elastomers, wie beispielsweise die Reißfestigkeit oder die maximale Dehnbarkeit, erhalten. Bevorzugt wird auch zumindest ein Fotoinitiator in einem Anteil von 0,5 phr bis 5 phr zugesetzt. Als Fotoinitiator können insbesondere α-Hydroxyalkylphenone, α-Aminoalkylphenone, Acylphosphinoxide, Benzoinether, Benzilketale, α-Dialkoxyacetophenone eingesetzt werden.

Zum besseren Verständnis der Erfindung wird diese anhand der nachfolgenden Figuren näher erläutert.

Es zeigen:
- Fig. 1: den Quellgrad von vernetzten, nicht erfindungsgemäßen XNBR-Latexfilmen über die Vernetzungszeit bei unterschiedlichen DEPEG-500 Konzentrationen;
- Fig. 2: den Quellgrad von vernetzten, nicht erfindungsgemäßen XNBR-Latexfilmen über die Vernetzungszeit bei unterschiedlichen GE-100-Konzentrationen;
- Fig. 3: den Quellgrad von vernetzten, nicht erfindungsgemäßen XNBR-Latexfilmen über die Vernetzungszeit bei unterschiedlichen SPE-Konzentrationen;
- Fig. 4: die Moduln (50% Dehnung) von vernetzten, nicht erfindungsgemäßen XNBR-Latexfilmen bei unterschiedlichen DEPEG-500-Konzentrationen;
- Fig. 5: die Moduln (50% Dehnung) von vernetzten, nicht erfindungsgemäßen XNBR-Latexfilmen (nicht steril / nicht gealtert) bei DEPEG-Typen mit unterschiedlichem Molekulargewicht;
- Fig. 6: die Moduln (50% Dehnung) von vernetzten, nicht erfindungsgemäßen XNBR-Latexfilmen (nicht steril / gealtert bei 70°C für 7 Tage) bei DEPEG-Typen mit unterschiedlichem Molekulargewicht;
- Fig. 7: die Reißfestigkeiten von UV vernetzten NR-Latexfilmen (nicht steril, nicht gealtert) bei unterschiedlichen Poly(mercaptopropylmethyl)siloxan-Konzentrationen (Synthesezeit: 3, 6 und 9h);
- Fig. 8: die Bruchdehnungen von UV vernetzten NR-Latexfilmen (nicht steril, nicht gealtert) bei unterschiedlichen Poly(mercaptopropylmethyl)siloxan-Konzentrationen (Synthesezeit: 3, 6 und 9h);
- Fig. 9: die Moduln (50% Dehnung) von UV vernetzten NR-Latexfilmen (nicht steril, nicht gealtert) bei unterschiedlichen Poly(mercaptopropylmethyl)siloxan-Konzentrationen (Synthesezeit: 3, 6 und 9h);
- Fig. 10: die Reißfestigkeiten von UV vernetzten NR-Latexfilmen (nicht steril, nicht gealtert) bei unterschiedlichen Poly(mercaptopropylmethyl)siloxan-Konzentrationen (Monomerkonzentration: 9 und 18% (w/v));
- Fig. 11: die Bruchdehnungen von UV vernetzten NR-Latexfilmen (nicht steril, nicht gealtert) bei unterschiedlichen Poly(mercaptopropylmethyl)siloxan-Konzentrationen (Mono-merkonzentration: 9 und 18% (w/v));
- Fig. 12: die Moduln (50% Dehnung) von UV vernetzten NR-Latexfilmen (nicht steril, nicht gealtert) bei unterschiedlichen Poly(mercaptopropylmethyl)siloxan-Konzentrationen (Monomerkonzentration: 9 und 18% (w/v)).

Sämtliche in der Beschreibung zitierten Normen beziehen sich auf die zum Anmeldezeitpunkt gegenständlicher Patentanmeldung gültige Fassung, sofern nichts anderes angegeben ist.

Die Erfindung bezieht sich auf ein Verfahren zum Herstellen eines Prophylaxeartikels.

Der Prophylaxeartikel ist bevorzugt ein Handschuh, insbesondere ein chirurgischer Handschuh (Operationshandschuh) oder ein Untersuchungshandschuh. Der Prophylaxeartikel kann aber beispielsweise auch ein Fingerling, ein Katheter, ein Kondom, ein (medizinischer) Ballon, ein Sauger, etc., sein. Generell ist der Prophylaxeartikel bevorzugt ein Tauchartikel, also ein Produkt, das mittels eines Tauchverfahrens hergestellt wird.

Im Folgenden wird nur mehr auf die Ausbildung des Prophylaxeartikels als Handschuh eingegangen. Die Ausführungen dazu können aber auch auf andere Elastomerartikel, insbesondere Tauchartikel die nach einem Tauchverfahren hergestellte werden, übertragen werden.

Der Handschuh umfasst ein Dienelastomer (Dienkautschuk), insbesondere ein carboxyliertes Dienelastomer, bzw. besteht aus diesem.

Das Elastomer der Elastomerschicht kann sowohl auf einem Natur- als auch auf einem Syntheselatex basieren. Diese können ausgewählt sein aus einer Gruppe umfassend oder bestehend aus Naturkautschuk (NR), Polyisopren-Latex (IR), Nitrilbutadienkautschuk-Latex (NBR), carboxylierter Nitrilbutadienkautschuk-Latex (XNBR), carboxylierter Butadienlatex (XBR), Chloropren-Latex (CR), Styrol-Butadien Latex (SBR), carboxylierte Latices hergestellt aus Polymer-Blends, sowie Mischungen daraus.

Insbesondere wird ein carboxylierter Nitrilbutadienkautschuk-Latex oder ein Polyisopren-Latex oder ein Naturkautschuk zur Herstellung der Elastomerschicht verwendet. Der Nitrilbutadienkautschuk-Latex weist bevorzugt einen Anteil an Acrylnitril zwischen 15 Gew.-% und 40 Gew.-%, insbesondere zwischen 20 Gew.-% und 35 Gew.-%, auf.

Der Prophylaxeartikel bzw. der Elastomerhandschuh wird bevorzugt nach einem Tauchverfahren hergestellt. Derartige Tauchverfahren sind aus dem Stand der Technik prinzipiell bekannt, sodass zu Einzelheiten dazu auf den einschlägigen Stand der Technik verwiesen sei.

Im Wesentlichen wird bei diesem Verfahren eine Tauchform (üblicherweise werden in der seriellen Fertigung mehrere Tauchformen verwendet) in ein Tauchbad eingetaucht. Die Tauchform weist dabei die Form des fertigen Produktes auf, also beispielsweise die Form einer Hand.

Im Tauchbad ist der jeweilige Elastomerlatex vorgelegt, der auf die Tauchform aufgetaucht werden soll.

Prinzipiell kann aber auch jede andere geeignete Form in den in dieser Beschreibung beschriebenen Verfahren verwendet werden, insbesondere wenn die Elastomerschicht nicht nach einem Tauchverfahren hergestellt wird. Die Elastomerschicht kann beispielsweise auch durch aufstreichen oder aufsprühen des Elastomerlatex auf eine Form hergestellt werden. Ebenso sind auch andere geeignete Verfahren der Aufbringung des Latex auf eine Form anwendbar.

Der Begriff Elastomerlatex wird in dieser Beschreibung dem üblichen Gebrauch in der Fachsprache entsprechend verwendet. Dementsprechend ist ein Elastomerlatex eine Dispersion aus unvernetzten oder vorvernetzten oder vernetzungsfähigen Polymermolekülen zur Herstellung eines Elastomers. Es können im Rahmen der Erfindung also auch vorvernetzte Elastomerlatices verarbeitet werden, wobei die Vorvernetzung insbesondere mittels dem in dieser Beschreibung genannten Vernetzungsmittel erfolgen kann, das ein mehrfach funktionelles Monomer und/oder Polymer ist, das dem (carboxylierten) Dienlatex zugesetzt und in diesem gelöst oder in diesem emulgiert bzw. dispergiert wird.

Es ist aber weiter möglich, dass der Elastomerlatex erst nach dem Aufbringen auf die Form, also der aufgebrachte Elastomerlatex, vernetzt wird.

Eine übliche Prozessroute eines Koagulations-Tauchverfahrens kann beispielsweise folgende Verfahrensschritte umfassen:
- waschen der Tauchform und entfetten mit einem organischen Lösungsmittel;
- vorwärmen der Tauchform;
- tauchen der Tauchform in ein erstes Tauchbad mit einem Koagulanten;
- Antrocknen der ersten aufgetauchten Schicht;
- tauchen der Tauchform in ein weiteres Tauchbad zur Ausbildung der Elastomerschicht;
- trocknen/vulkanisieren (vernetzen);
- abziehen des Tauchartikels von der Form.

Wie im Nachfolgenden noch näher ausgeführt wird, kann anstelle der thermischen Vernetzung der Elastomermoleküle auch eine photochemische Vernetzung mittels UV-Licht, gegebenenfalls nach Zusatz eines Fotoinitiators, durchgeführt werden. Der Fotoinitiator kann eine handelsüblicher Fotoinitiator sein und in üblichen Konzentrationen zugesetzt werden. Es sei hierzu auf die eingangs genannten US 2014/0096307 A1 und US 2014/0096308 A1 verwiesen, die in dem Umfang der Fotoinitiatoren und deren Konzentrationen im Latex zu gegenständlicher Beschreibung gehören.

Für den Fall, dass der Elastomerhandschuh mehrschichtig ausgebildet wird, können weitere Schichten aus dem ersten Elastomerlatex oder aus einem anderen Elastomerlatex oder einem anderen Polymer aufgetaucht oder generell aufgebracht werden. Beispielsweise kann als letzte Schicht eine Polymerschicht aufgetaucht werden, die nach dem Abziehen des Handschuhs von der Tauchform durch das dabei erfolgende Wenden des Handschuhs an die Innenseite des Handschuhs gelangt. Derartige Polymerschichten können beispielsweise als Gleitschichten ausgebildet sein, um die Anziehbarkeit des Elastomerhandschuhs zu verbessern.

Der Elastomerhandschuh kann also ein- oder mehrschichtig ausgebildet sein, wobei die einzelnen Schichten aus zueinander unterschiedlichen Werkstoffen oder aus den gleichen Werkstoffen bestehen können. Es ist auch möglich, dass zwei oder mehr Schichten des Elastomerhandschuhs aus dem gleichen Werkstoff und eine oder mehr Schichten aus einem dazu unterschiedlichen Werkstoff bestehen.

Da all dies an sich bekannt ist, soll nicht weiter darauf eingegangen werden.

Unter Werkstoffen werden dabei in dieser Beschreibung Elastomere und Polymere verstanden, wobei aber der Elastomerhandschuh zumindest eine Schicht aus einem Elastomer aufweist.

Die Begriffe Vulkanisation und Vernetzung werden in dieser Beschreibung synonym verwendet.

Zur Vernetzung des (carboxylierten) Dienelastomer-Latex wird diesem, d.h. insbesondere dem Tauchbad zur Herstellung der zumindest einen Schicht aus dem (carboxylierten) Dienelastomers, ein Vernetzungsmittel zugesetzt. Daneben kann der Dienelastomer-Latex bzw. das Tauchbad zumindest ein weiteres Additiv, wie beispielsweise zumindest einen Emulgator, zumindest ein Alterungsschutzmittel, zumindest einen Farbstoff, zumindest ein Antiozonat, aufweisen, wie sie an sich für die Herstellung von Tauchartikeln bekannt sind. Der Gesamtanteil an diesen Additiven kann zwischen 0,1 phr und 10 phr, bezogen auf die Gesamtzusammensetzung des Dienelastomer-Latex bzw. des Tauchbads, betragen.

Dem (carboxylierten) Dienelastomer-Latex wird ein Vernetzungsmittel auf polymerer Basis zugesetzt und in dem (carboxylierten) Dienelastomer-Latex aufgelöst. Die Konzentration an Vernetzungsmittel kann zwischen 1 phr und 15 phr, insbesondere zwischen 1 phr und 7,5 phr, betragen.

In der bevorzugten Ausführungsvariante des Verfahrens werden keine weiteren Vernetzungsmittel verwendet, d.h. dass als Vernetzungsmittel ausschließlich das in dem (carboxylierten) Dienelastomer-Latex lösliche Polymer verwendet wird. Es kann jedoch, wie voranstehend bereits ausgeführt, zumindest ein Fotoinitiator zugesetzt werden.

Der Begriff "Polymer" im Sinne dieser Beschreibung umfasst dabei generell Moleküle ab zwei Monomereinheiten, also Moleküle ab Dimeren.

Generell umfasst im Rahmen der Erfindung der Begriff "Polymer" auch Oligomere.

Als Vernetzungsmittel wird ein mercaptofunktionelles Siloxanpolymer verwendet.

Gemäß einer Ausführungsvariante des Verfahrens kann vorgesehen sein, dass als mercaptofunktionelles Siloxanpolymer ein mercaptofunktionelles Siloxanhomopolymer oder ein Copolymer des mercaptofunktionellen Siloxanhomopolymers mit einem Acrylsiloxan verwendet wird. Insbesondere wird ein mercaptofunktionelles Siloxanhomopolymer mit der Strukturformel 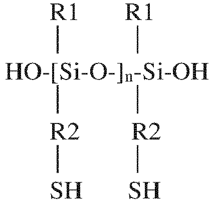 eingesetzt, wobei R1 für eine erste Einheit ausgewählt aus einer ersten Gruppe bestehend aus -CH3, -OH, -C₂H₅, -C₃H₇, aromatische Gruppen, R2 für eine zweite Einheit ausgewählt aus zweiten Gruppe bestehend aus -CH2, C2H4, C3H6; -(CH₂)₁₁-, aromatische Gruppen, -CH2-Aromat, steht, und/oder ein Acrylsiloxan mit der Strukturformel 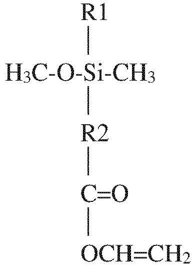 eingesetzt, wobei R1 für eine erste Einheit ausgewählt aus einer ersten Gruppe bestehend aus -OH, -CH₃, -C₂H₅, -C₃H₇, aromatische Gruppen, R2 für eine zweite Einheit ausgewählt aus zweiten Gruppe bestehend aus -CH₂, C₂H₄, C₃H₆; aromatische Gruppen, steht.

Als mercaptofunktionelles Siloxan kann auch das nachstehende Dimer eingesetzt werden.

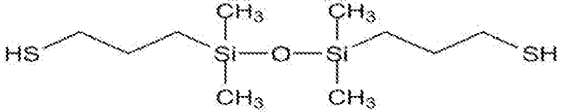

Nach einer besonders bevorzugten Ausführungsvariante des Verfahrens ist das mercaptofunktionelle Siloxanhomopolymer ausgewählt ist aus einer Gruppe bestehend aus Poly(mercaptomethylpropyl)siloxan, Poly(mercaptomethylpropyl)siloxan, Poly(mercaptomethylmethyl)siloxan, Poly(mercaptoethylmethyl)siloxan, Poly(mercaptomethylethyl)siloxan, Po-ly(mercaptopropylmethyl)siloxan, Poly(mercaptomethylbenzyl)siloxan, Po-ly(mercaptopropylbenzyl)siloxan, Poly(mercaptoethylbenzyl)siloxan und/oder das Copolymer des mercaptofunktionellen Siloxanhomopolymers mit einem Acrylsiloxan ausgewählt ist aus einer Gruppe bestehend aus Poly(mercaptomethylpropyl-co-acryloxymethylpropyl)siloxan, Poly(mercaptomethylmethyl-co-acryloxymethylpropyl)siloxan, Po-ly(mercaptomethylmethyl-co-acryloxypropylmethyl)siloxan, Po-ly(mercaptomethylmethyl-co-acryloxypropylethyl)siloxan, Po-ly(mercaptomethylmethyl-co-acryloxyethylpropyl)siloxan, Po-ly(mercaptomethylmethyl-co-acryloxymethylmethyl)siloxan, Po-ly(mercaptomethylmethyl-co-acryloxypropyl)siloxan, Poly(mercaptomethylmethyl-co-acryloxyethyl)siloxan, Poly(mercaptomethylmethyl-co-acryloxymethyl)siloxan, Poly(mercaptopropylmethyl-co-acryloxymethylpropyl)siloxan.

Der Anteil des mercaptofunktionellen Siloxanpolymers an dem Copolymer des mercaptofunktionellen Siloxanpolymers mit einem Acrylsiloxan kann ausgewählt sein aus einem Bereich von 20 Gew.-% bis 99 Gew.-%, insbesondere aus einem Bereich von 20 Gew.-% bis 80 Gew.-%.

Die Schichtdicke der Elastomerschicht kann zwischen 30 µm und 500 µm betragen.

Generell kann der (carboxylierte) Dienelastomer-Latex einen Feststoffgehalt an (carboxyliertem) Dienelastomer zwischen 10 drc (dry rubber content) und 60 drc aufweisen.

Es ist weiter von Vorteil, wenn der pH-Wert des (carboxylierten) Dienelastomer-Latex auf einen Wert von größer/gleich 9 eingestellt wird. Dazu kann beispielsweise eine wässerige KOH-Lösung (lGew.-% bis 5 Gew.-%) verwendet werden. Generell können dazu geeignete basische Substanzen, wie Laugen, verwendet werden.

In einer bevorzugten Ausführungsvariante des Verfahrens erfolgt die Vernetzung der (carboxylierten) Dienelastomermoleküle thermisch, insbesondere während der Trocknung der (aufgetauchten) Schicht aus dem (carboxylierten) Dienelastomer-Latex. Die Temperatur kann dabei zwischen 90 °C und 140 °C betragen. Die Vernetzung kann während einer Zeitspanne zwischen 5 Minuten und 20 Minuten erfolgen.

Es kann ein Vernetzungsmittel eingesetzt werden, das ein Molekulargewicht zwischen 170 g/mol und 4000 g/mol, insbesondere zwischen 170 g/mol und 1700 g/mol (polymere, wasserlösliche Verbindungen gemäß DIN 55672-3:2007-08 (GPC)) oder über die Viskosität von flüssigen Polymeren gemäß DIN 51 562-1) aufweist. Es ist damit auch möglich, den (50%-) Modul des Elastomerhandschuhs auf einen gewünschten Wert einzustellen. Das Modul des Elastomerhandschuhs kann über die Kettenlänge des Vernetzungsmittels eingestellt werden.

Mit dem Verfahren kann ein Prophylaxeartikel, insbesondere Handschuh, hergestellt werden, umfassend eine Schicht aus einem (carboxylierten) Dienelastomer, wobei die (carboxylierten) Dienelastomermolekülketten des (carboxylierten) Dienelastomers kovalent über organische Moleküle vernetzt sind.

Die nach dem Verfahren hergestellten Elastomerhandschuhe weisen eine gute Hautverträglichkeit auf. Anhand von durchgeführten Untersuchungen konnte kein Hautreizungs- und kein Sensibilisierungspotential festgestellt werden.

Im Zuge der Erprobung des Vernetzungsverfahrens wurden u.a. folgende Experimente durchgeführt. Bei diesen handelt es sich nur um ausgewählte Beispiele, da die Wiedergabe sämtlicher Experimente den Rahmen dieser Beschreibung sprengen würde.

Im Folgenden sind die Versuchsergebnisse für nicht erfindungsgemäße Ausführungen des Verfahrens mit mehrfachfunktionellen Monomeren und/oder Polymeren als Vernetzungsmittel wiedergegeben. In Tabelle 1 sind die hierfür verwendeten Edukte zusammengefasst.

**Tabelle 1: verwendete Materialien**

| *Name* | *Funktion* | *Beschreibung* |
|---|---|---|
| Nipol LX556 ZEON Corporation (JPN) | Latex | 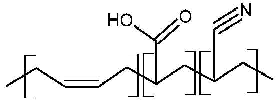 |
| BST8502N PolyLac 582N | | XNBR Trockenkautschukgehalt: 45,2% pH-Wert: 8 bis 8.8 |
| SPE, Epoxy-Sorbit CVC Thermo-set Specialities | Polymeres Vernetzungsmittel | 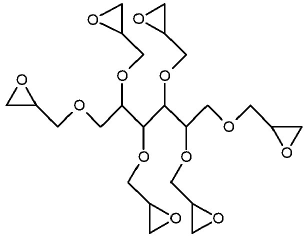 |
| | | Sorbitol polyglycidyl ether (ERISYS GE 60) |
| GE100 Raschig | Polymeres Vernetzungsmittel | 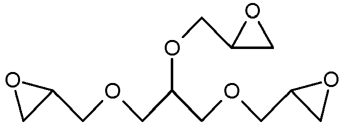 |
| | | Glycerol glycidyl ether |
| DEPEG Sigma-Aldrich (USA) PolyScience (USA) | Polymeres Vernetzungsmittel | 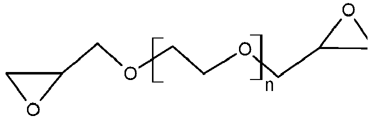 |
| | | Diepoxy-terminiertes Polyethylenglykol |
| | | DEPEG-200 Mn=200 |
| | | DEPEG-500 Mn=500 |
| | | DEPEG-1000 Mn=1000 |

### Herstellung der Latexmischungen, Tauchung und Vernetzung

Das wasserlösliche Vernetzungsmittel wurde in unterschiedlichen Konzentrationen (0,5 bis 1,5phr) der Latexmischung (pH = 10, ∼25 drc.) zugegeben. Anschließend wurde die Mischung mit einem Alterungsschutzmittel (0,5 phr bis 2 phr Ralox) versetzt und etwa 15min bei Raumtemperatur gerührt. Anschließend wurden die Filme mittels dem voranstehend beschriebenen Koagulationstauchverfahren hergestellt und die Filme bei 100°C für 15min getrocknet. Es wurde keine Vorvernetzung bzw. Latexreifung benötigt. Die Vernetzung fand während der Trocknung der Filme bei 100°C statt.

Die Latexmischung kann während des Tauchprozesses leicht mit Hilfe eines Magnetrührers gerührt werden. Dies trifft generell auf das in dieser Beschreibung beschriebene Verfahren zu.

Nachfolgende Reaktionen liegen der thermischen Vernetzung mit Monomeren und/oder polymeren Epoxidvernetzern zu Grunde. Von Vorteil ist im Vorfeld die Einstellung des pH-Werts der Latexmischung, beispielsweise mit 1 Gew.-%iger KOH auf pH=10, da die Reaktion bei höheren pH-Werten katalysiert wird.

Reaktion eines carboxylierten Elastomers mit einem Epoxid 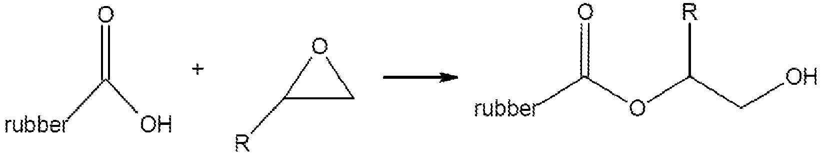 Sauer und basisch katalysierte Ringöffnung von Epoxiden.

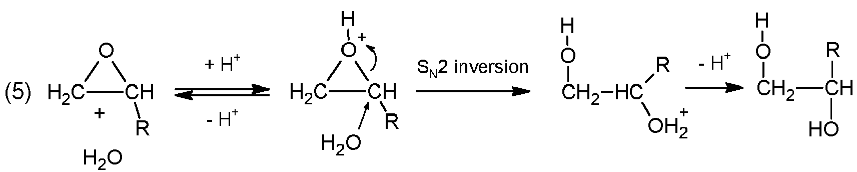

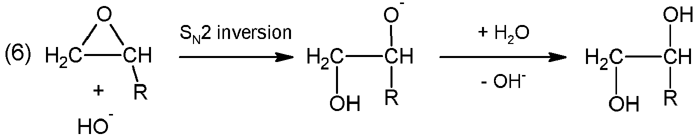

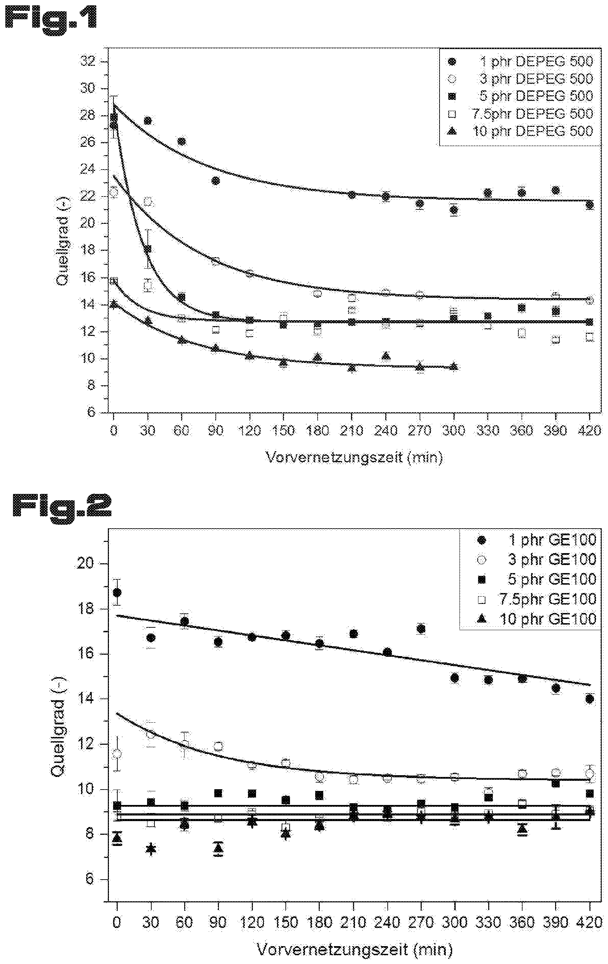

Die erfolgreiche Vernetzung von XNBR-Latex bei Zugabe von ausgewählten wasserlöslichen polymeren Vernetzungsmittels wurde mittels Gleichgewichtsquellung in Chloroform (bestimmt nach: (1) Macromolecules 2008, 41, 4717-4729, (2) J. Appl. Polym. Sci. 129(5), 2735-2743 bzw. (3) Zaborski, M.; Kosmalska, A.; Gulinski, J. Kautsch. Gummi Kunstst. 2005, 58, 354) nachgewiesen. Die Ergebnisse sind in den Fig. 1 bis 3 dargestellt. Dabei sind auf den Abszissen die Vernetzungszeit in Minuten und auf den Ordinaten der Quellgrad dargestellt. Die Vernetzungsdichte steigt hierbei mit zunehmender Vernetzungszeit und Vernetzungsmittelkonzentration wobei die Reaktivität der Vernetzungsmittel von DEPEG-500 < SPE < GE100 zunimmt.

Neben der Gleichgewichtsquellung wurde die Vernetzung von XNBR-Latex bei Zugabe von ausgewählten wasserlöslichen polymeren Vernetzungsmittel auch mittels Zugprüfung nachgewiesen.

Bei Einsatz von DEPEG-500 können ab einer Konzentration von 5 phr mechanische Festigkeiten im Bereich von 22 ± 2 MPa beobachtet werden. Bei geringeren Konzentrationen (0,5 bis 3 phr) wird eine geringe Vernetzungsdichte erzielt und die Reißfestigkeiten liegen unter 10 MPa. Eine Erhöhung der Vernetzerkonzentration auf 7,5 phr bringt eine weitere Erhöhung der Festigkeiten auf bis zu 35 ± 2 MPa. Bevorzugt wird daher eine Konzentration von 5 phr bis 7,5 phr.

Sehr gute mechanische Festigkeiten und Alterungs- bzw. Gamma-Beständigkeiten wurden auch mit DEPEG-200 in einem Konzentrationsbereich zwischen 3 phr und 7,5 phr beobachtet (nicht steril/ nicht gealtert: 26 MPa - 40 MPa; nicht steril/gealtert: 37 MPa -26 MPa; steril/nicht gealtert: 28 MPa -24MPa; steril/ gealtert: 25 MPa -35MPa).

Da ähnliche Ergebnisse auch mit anderen mehrfachfunktionellen Monomeren bzw. polymeren Vernetzungsmitteln erzielt wurden, wird generell eine Konzentration von 1 phr bis 7,5 phr mehrfachfunktionelle Monomeren und/oder polymere Vernetzungsmittel im Latex bevorzugt.

Weiter wird eine ausgezeichnete Heißluftalterungs- (7 Tage Lagerung bei 70°C) und Gamma-Beständigkeit (25 kGy) beobachtet.

Generell sei angemerkt, dass im Zuge der Tests des Prophylaxeartikels die Sterilisation durch Gamma-Strahlung mit einer Co-60-Quelle und einer Bestrahlungsdosis von 25kGy erfolgen kann. Die Alterung kann generell durch Heißluftalterung bei 70°C im Umlufttrockenofen über 7Tage erfolgen.

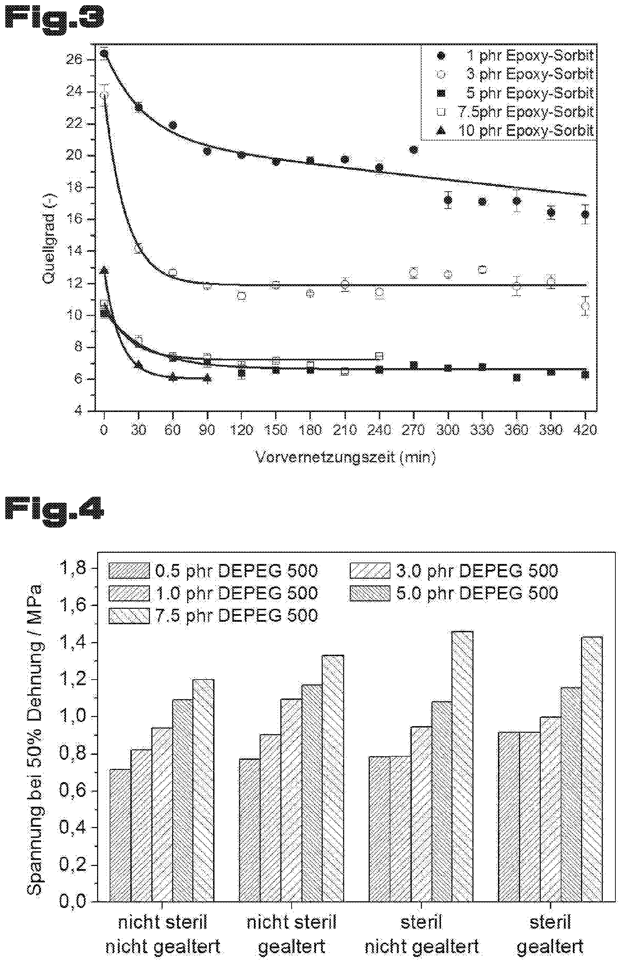

Zusätzlich liegt der Spannungswert bei 50% Dehnung auch bei hohen Reißfestigkeiten im Bereich von 1,2 bis 1,4 MPa und wird insbesondere bei Einsatz von 5 phr Vernetzungsmittel auch nach Heißluftalterung und Gammasterilisation kaum erhöht. Dies ist vor allem für die Herstellung von Operationshandschuhen von Vorteil, da ein geringer Spannungswert bei 50 % Dehnung ein Kriterium für einen angenehmen Tragekomfort darstellt. Die Ergebnisse der Messung der 50 % Dehnung sind in Fig. 4 dargestellt. Die Balken sind darin in Fünfergruppen angerordnet, wobei innerhalb jeder Fünfergruppe die Balken für eine Konzentration an DEPEG-500 von links nach rechts von 0,5 phr, 1,0 phr, 3,0 phr, 5,0 phr und 7,5 phr stehen. Die Fünfergruppen selbst stehen von links nach rechts für nicht sterile und nicht gealterte, nicht sterile und gealterte, sterile und nicht gealterte sowie sterile und gealterte Proben. Auf der Ordinate sind die 50 % Moduln in MPa angegeben.

Analog zur Vernetzung mit DEPEG-500 wurden auch bei Einsatz von SPE (Epoxy-Sorbit) sehr gute mechanische Eigenschaften (auch nach Gamma-Sterilisation) bei höheren Konzentrationen (7,5 phr) nachgewiesen. Bei einer Konzentration von 7,5 phr SPE wurden Werte für die mechanischen Eigenschaften zwischen 12 MPa und 32 MPa gemessen (nicht steril/ nicht gealtert: 30 MPa - 32 MPa; nicht steril/gealtert: 12 MPa -14 MPa; steril/nicht gealtert: 30 MPa -32MPa; steril/ gealtert: 13 MPa -15MPa). Bei einer Konzentration von DEPEG-500 zwischen 0,5 phr und 1,0 phr wurden hingegen nur Werte von maximal ca. 5 MPa gemessen. DEPEG-500 wird deshalb bevorzugt in einer Menge von 5 phr bis 7,5 phr eingesetzt.

Bei Verwendung von SPE als wasserlösliches polymeres Vernetzungsmittel wird zusätzlich eine ausgeprägte Erhöhung des Spannungswertes bei 50 % Dehnung beobachtet, was nachteilig für den Tragekomfort des Elastomerhandschuhs ist. Bei 7,5 phr SPE werden Werte im Bereich von 1,6 bis 1,8 MPa erhalten. SPE wird daher vorzugsweise in einer Konzentration von 0,5 phr bis 5 phr eingesetzt.

Bei Einsatz von GE100 als Vernetzungsmittel werden bereits bei geringen Konzentrationen (1 und 3phr) sehr gute mechanische Festigkeiten erzielt, die im Bereich von 20 bis 27 MPa liegen. Mit höheren Vernetzungsmittelkonzentrationen (7,5 phr) wird eine weitere Erhöhung der Reißfestigkeiten beobachtet (37 ± 2MPa). Bei einer Konzentration von 5 phr werden Werte zwischen 22 MPa und 40 MPa erhalten (nicht steril/ nicht gealtert: 35 MPa - 40 MPa; nicht steril/gealtert: 32 MPa -35 MPa; steril/nicht gealtert: 36 MPa -38MPa; steril/ gealtert: 22 MPa -23MPa). Die vernetzten XNBR-Latexfilme zeichnen sich durch eine sehr gute Gamma-Beständigkeit aus.

Zusammenfassend kann aus den Ergebnissen geschlossen werden, dass hohe Reißfestigkeiten (30 ± 2MPa) und Gamma-Beständigkeiten (nach Gammasterilisation: 30 ± 2MPa) mit allen drei untersuchten Vernetzungsmitteln erzielt worden sind. Hinsichtlich Beständigkeit gegenüber einer Heißluftalterung oder einem geringen Modul bei 50 % Dehnung weist DEPEG-500 klare Vorteile gegenüber GE-100 und SPE auf.

Basierend auf diesen Ergebnissen wurde in weiteren Untersuchungen der Modulwert der vernetzten XNBR-Latexfilme durch das Molekulargewicht des epoxid-terminierten Polyethylenglykolderivats (DEPEG) gezielt eingestellt. Mit niedrigerem Molekulargewicht wird einerseits eine sehr hohe Festigkeit (bis zu 40MPa) erreicht, während der Modul ansteigt. Dies ist vor allem für die Herstellung von Untersuchungshandschuhen interessant, wo hohe Festigkeiten im Vordergrund stehen und der Modul (auf Grund der Schichtdicke) nur eine untergeordnete Rolle spielt. Bei XNBR-Filmen, die mit DEPEG-500 (mittleres Molekulargewicht) vernetzt worden sind, erhält man zwar etwas geringere Festigkeiten, aber die Modulwerte sind wesentlich geringer. Diese Variante eignet sich mehr für die Herstellung von Operationshandschuhen, wo das Hauptaugenmerk auf einen niedrigen Modul liegt.

Liegt das Molekulargewicht des Vernetzungsmittels jedoch im Bereich von 1.000 g/mol, kann zwar der 50 % Modulwert unter 1MPa gebracht werden, aber die entsprechenden Reißfestigkeiten liegen auch unter 15MPa. Die Ergebnisse zeigen daher, dass über die Kettenlänge des Vernetzungsmittels eine Balance zwischen Reißfestigkeit und Modul eingestellt werden kann. Es werden daher die voranstehend angeführten Kettenlängen der polymeren Vernetzungsmittel bevorzugt.

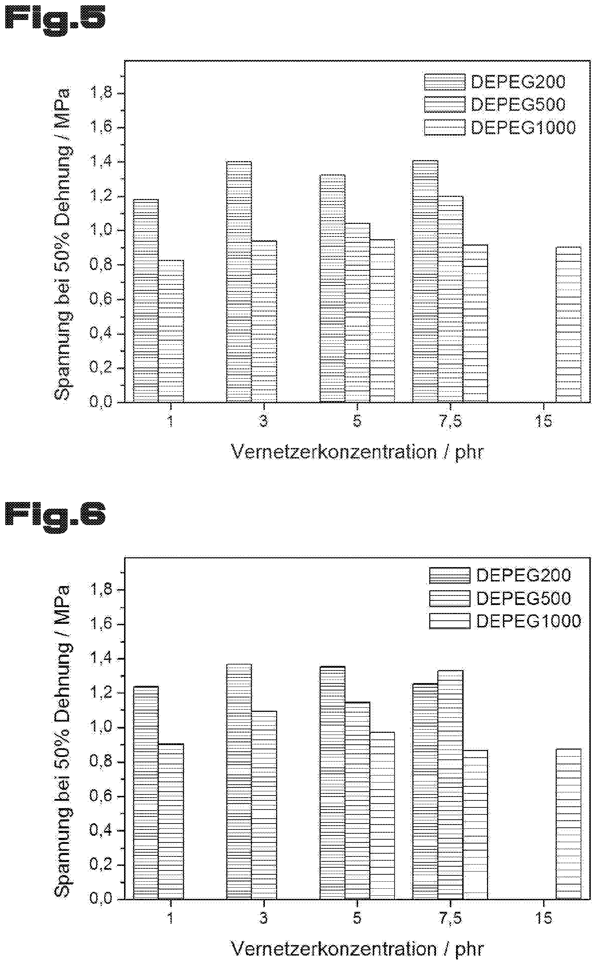

Die Messergebnisse dieser Untersuchung sind in den Fig. 5 und 6 dargestellt. Dabei sind auf den Abszissen die Konzentration an Vernetzungsmittel in phr und auf den Ordinaten die gemessenen Spannungen bei 50 % Dehnungen in MPa aufgetragen.

In weiteren Untersuchungen wurde PolyLac 582N als weitere alternative Latextype bei unterschiedlichen pH-Werten mit 5 phr DEPEG-200 vernetzt. Die Ergebnisse zeigen deutlich, dass eine erfolgreiche Vernetzung von PolyLac 582N gelingt.

Im Folgenden werden ausgewählte Beispiele zur photochemischen Vernetzung von Elastomerlatices wiedergegeben. In Tabelle 2 sind die hierfür verwendeten Edukte zusammengefasst.

**Tabelle 2: verwendete Materialien**

| *Name* | *Funktion* | *Beschreibung* |
|---|---|---|
| Naturkautschuk-Latex | Latex | NR High ammonia, 60% drc |
| Isoprenkautschuk -Latex Kraton | Latex | 60% drc |
| 3-Mercaptopropyltrimethoxysilan ABCR | Monomer für Synthese des polymeren Vernetzers | 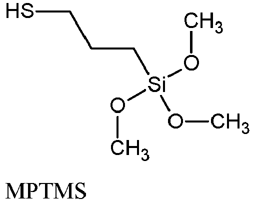 |
| 3-Mercaptopropylmethyldimethoxysilan ABCR | Monomer für Synthese des polymeren Vernetzers | 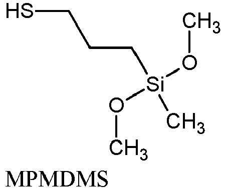 |
| 3-Mercaptomethylmethyldimethoxysilan ABCR | Monomer für Synthese des polymeren Vernetzers | 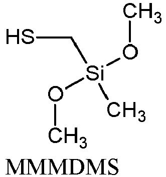 |
| 3-Acryloxypropylmethyldimethoxysilan ABCR | Monomer für Synthese des polymeren Vernetzers | 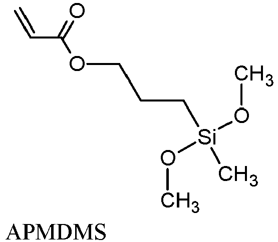 |
| Lucirin TPO-L BASF | Photoinitiator | 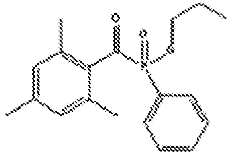 |
| Tween 20 | Emulgator | 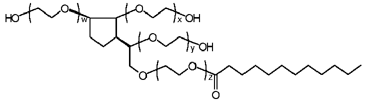 |
| Ionol LC | Antioxidant | 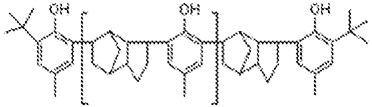 |

Die Herstellung des polymeren Siloxanvernetzungsmittels kann wie folgt erfolgen.

0,1 M HCl (aq.) und Ethanol werden vorgelegt, auf 50 °C erhitzt und mit einem kontinuierlichen N₂-Strom gespült. Anschließend werden die entsprechenden Siloxanmonomere (siehe Tabelle 2) in ausgewählten Konzentrationen von 5 % (w/v) und 40 % (w/v) hinzugefügt.

Nach 3 bis 9 Stunden bei 50°C wird die Reaktion durch Abkühlung gestoppt und das ölige Produkt mit deionisiertem Wasser gewaschen und mit Chloroform extrahiert. Das Lösungsmittel wird im Abschluss unter Vakuum abgezogen und das Produkt unter N₂-Atmosphäre gelagert.

Nachfolgend sind die Reaktionsschemata der synthetisierten Homo- und Copolymere aufgelistet.

### Synthese von Poly(mercaptopropyl)siloxan:

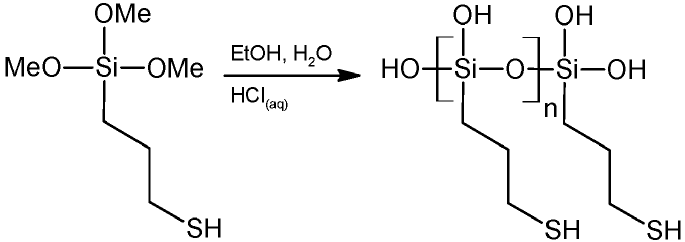

### Synthese von Poly(mercaptomethylmethyl)siloxan:

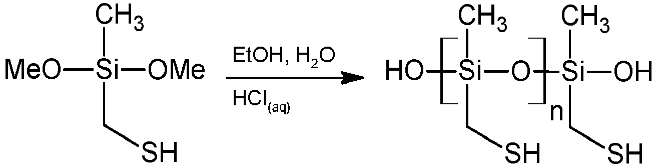

### Synthese von Poly(mercaptopropylmethyl)siloxan

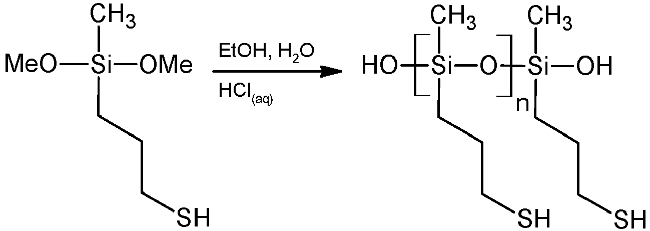

### Synthese von Poly(mercaptopropylmethyl-co-acryloxypropylmethyl)siloxan

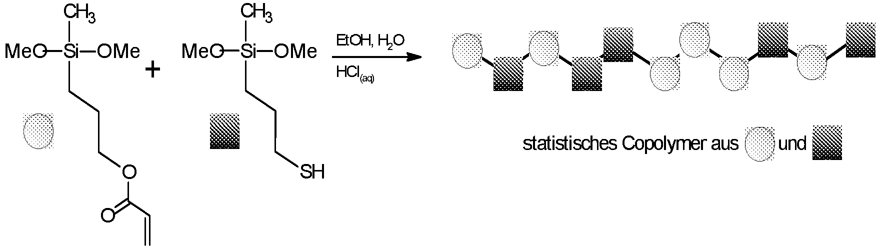

Die Molekulargewichtsverteilung der Siloxane wurde mittels Gelpermeationschromatographie (Universalkalibration mit Polystyrolstandards) bestimmt. Folgende Ergebnisse wurden erhalten:
- Poly(mercaptopropylmethyl)siloxan (3 Stunden - Reaktionszeit)
   Molekulargewicht: 200 g/mol - 700 g/mol (2 bis 5 Einheiten)
- Poly(mercaptopropylmethyl)siloxan (9 Stunden - Reaktionszeit)
   Molekulargewicht: 200 - 1.400 g/mol (2 bis 10 Einheiten)
- Poly(mercaptopropylmethyl-co-acryloxypropylmethyl)siloxan (3 Stunden Reaktionszeit)
   Molekulargewicht: 200 - 1.300 g/mol

### Herstellung der Latexfilme und UV-Vernetzung mit polymeren Siloxanvernetzungsmitteln

Die synthetisierten polymeren Vernetzungsmittel werden in unterschiedlichen Konzentrationen (1 bis 4 phr) mit Lucirin TPO-L (1phr) in deionisiertem Wasser mit Tween 20 (0,1 phr) emulgiert und anschließend dem NR-Latex (40 drc.) zugegeben. Die Latexmischung wird mit einem Alterungsschutzmittel (0,5 phr Ionol LC) versetzt und zwei Stunden bei Raumtemperatur gerührt. Anschließend werden die Filme mittels nachfolgendem Koagulationstauchverfahren hergestellt:
- Waschen der Keramikformen und Entfetten mit Aceton
- Vorwärmen der Keramikformen für mindestens 10 min im Trockenschrank bei 120°C
- Tauchen der Form für 30 s in das Koagulationsbad bei 70°C
- Trocknen der Form für mindestens 1 min im Trockenschrank bei 120°C
- Tauchen der Form in die NR-Latexmischung für 20s
- Trocknen für 20 min im Trockenschrank bei 120°C
- Abziehen des Films

Die UV-Vernetzung der NR-Latexfilme erfolgte im Zuge einer UV-Belichtung der getrockneten Filme (Post-Curing) mit einem UV-Strahler von Fusion UV Systems Inc. Die UV-Belichtung erfolgte unter Luft mit einem Ga-dotierten Hg-Strahler bei einer Lampenleistung von 60% und einer Bandgeschwindigkeit von 3,5 m/min. Bei dreimaligem Durchlauf entspricht die Strahlungsdosis 15,6 J/cm².

Es sei darauf hingewiesen, dass die angegebenen Parameter nicht beschränkend zu verstehen sind, sondern lediglich einen Weg aufzeigen, die Prophylaxeartikel beispielsweise im Labormaßstab herzustellen. In der großtechnischen Anwendung können geringfügig andere Parameter erforderlich sein, die jedoch anhand weniger Versuche aufgefunden werden können.

Nachfolgender Reaktionsmechanismus liegt der photochemischen Vernetzung mit polymeren Siloxanvernetzungsmitteln zu Grunde.
(1) Initiation: 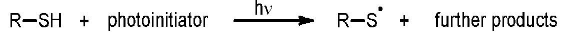
(2) Propagation: 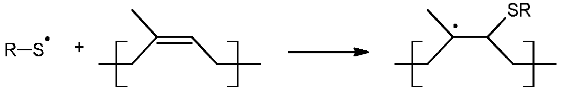 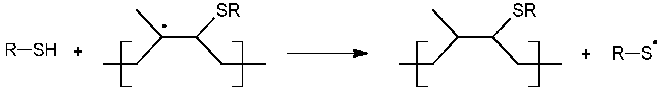
(3) Termination:

   R-S^{•} + R-S^{•} → R-S-S-R

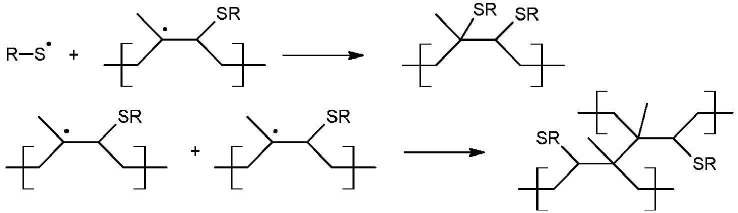

Generell können folgende Paramter für die UV-Vernetzung verwendet werden:
IR-Latices - Parameter für UV-Vorvernetzung im Fallfilmreaktor: Strahlerleistung bei 800 W -1000 W (800 W ergibt einen mittleren Strahlungsfluss von ∼ 500 mW/cm₂), zweifacher Belichtungsdurchgang, Fördergeschwindigkeit (Latexmischung) bei 1,1 1/min bis 1,5 1/min, Feststoffgehalt (Latex) bei 40 % drc., Fotoinitiatorkonzentration bei 0,5 phr bis 2phr, Thiolkonzentration bei 0,5 phr bis 2phr.
NR-Latices - Parameter für UV-Vorvernetzung im Fallfilmreaktor: Strahlerleistung bei 2000 W- 3500 W (3000 W ergibt einen mittleren Strahlungsfluss von ∼ 1690 mW/cm²) zweifacher Belichtungsdurchgang, Fördergeschwindigkeit (Latexmischung) bei 1,1 1/min bis 1,5 1/min, Feststoffgehalt (Latex) bei 40 % drc., Fotoinitiatorkonzentration bei 0,5 phr bis 2phr, Thiolkonzentration bei 1 phr bis 5phr.

Generelle Parameter für UV-Nachvernetzung: Restfeuchtegehalt der Filme bevorzugt unter 20 %. Nachdosierung von 0,5 phr bis 5 phr Fotoinitiator und 1 phr bis 7,5 phr Thiol, Bestrahlungsdosis zwischen 1 J/cm² und 25 J/cm² (240 nm - 420 nm Wellenlängenbereich).

Die Belichtung erfolgt vorzugsweise unter Luft mit einem Ga-dotierten Hg-Strahler.

Die Struktur der polymeren Vernetzungsmittel wurden mittels FT-IR Spektroskopie und mittels Thermogravimetrie (TGA) bestimmt. In den FT-IR Spektren der mercaptofunktionellen Siloxanhomopolymere Poly(mercaptopropyl)siloxan, Poly(mercaptomethylpropyl)siloxan und Poly(mercaptomethylmethyl)siloxan kann eine signifikante Verringerung der Si-O-CH₃ Bande bei ca. 2830 cm⁻¹ sowie die Bildung von OH-Gruppen (ca. 3370 cm-1) beobachtet werden, die auf eine erfolgreiche Kondensationsreaktion der Siloxanmonore (Alkoxysilanmonomere) rückschließen lassen. Weiter weist die Verbreiterung der Si-O-Bande bei ca. 1060 cm⁻¹ auf die Bildung einer polymeren Verbindung hin. Die charakteristische SH-Bande (ca. 2558 cm-1) ist nur schwach ausgeprägt, da die Infrarotbanden von Thiolgruppen generell eine sehr geringe Intensität aufweisen. Im FT-IR-Spektrum des Copolymers Poly(mercaptopropylmethyl-co-acryloxymethylpropyl)siloxan sind zusätzlich die charakteristischen IR-Banden der Acrylatgruppe (C=O-Banden bei 1727 cm⁻¹ und C=C-Banden bei 1637 und 1622 cm⁻¹) nachweisbar.

Im Zuge der TGA-Untersuchungen konnte gezeigt werden, dass die Homo- und Copolymere je nach Struktur bis zu einem Temperaturbereich von 240 °C bis 270°C stabil sind und dann einen mehrstufigen Abbau zeigen.

Um die Reaktivität der polymeren Vernetzungsmittel zu bestimmen wurde eine 2 Gew.-% Lösung Polyisoprenstandard in Chloroform hergestellt und mit 1phr Lucirin TPO-L und 5 phr des entsprechenden Thiols versetzt. Die Mischung wurde auf CaF₂-Plättchen gerakelt, das Lösungsmittel abgedampft und die dünnen Schichten anschließend mit einer UV-Lampe (OmniCure Series 1000; high pressure lamp, full power: 100 W von EXFO) belichtet. Nach unterschiedlichen Belichtungszeiten wurden IR-Spektren aufgenommen und die Abnahme der normierten C=C-Bande (835 cm⁻¹) über die Belichtungszeit aufgenommen. Im Vergleich zu dem kommerziell verfügbaren hochmolekularen Thiol Dipentaerythritolhexa(3-mercaptopropionat) (THIOCURE® Di-PETMP, Bruno Bock Thiochemicals) weisen die Siloxanpolymere eine wesentlich höhere Reaktivität in der Vernetzung auf. Während bei Einsatz von THIOCURE® Di-PETMP die relative Abnahme der C=C Banden nach einer Belichtungszeit von 150s etwa 5% beträgt kann bei Einsatz von Poly(mercaptopropylmethyl)siloxan eine Abnahme im Bereich von 12% erzielt werden.

Die Reaktivität der polymeren Vernetzer in der UV initiierten Thiol-En Reaktion wurde in weiteren Experimenten bestätigt. Dazu wurden die Vernetzungsmittel (1 phr) zusammen mit einem Photoinitiator (1 phr Lucirin TPO-L) in eine Polyisoprenstandardlösung (2 Gew.-% in Chloroform) gemischt und anschließend dünne Filme (40 µm) gerakelt, getrocknet, strukturiert belichtet und in Chloroform entwickelt. Ähnlich wie bei einem Negativresist werden durch die Thiol-En Reaktion die belichteten Bereiche der Schicht vernetzt und in der anschließenden Entwicklung in Chloroform konnten nur die nicht belichteten Bereiche gelöst und entfernt werden. Dieses Experiment wurde mit einem Mask Aligner bei einer sehr geringen Belichtungsdosis (∼20 mW/cm², 80 s) durchgeführt, um den Einfluss der direkten C-C-Verknüpfung der Polymerketten durch die Photoinitiatorradikale möglichst gering zu halten. Die Ergebnisse zeigen, dass bereits eine geringe Konzentration (1 phr) der polymeren Vernetzer ausreicht, um eine sehr hohe ortsaufgelöste Vernetzung des Polyisoprenstandards zu erhalten. Die Ergebnisse bestätigen somit die hohe Reaktiviät und Effizienz der synthetisierten polymeren Vernetzer in der Thiol-En Reaktion.

Auf Grund seiner chemischen Struktur (hohe Konzentration an freien Si-OH-Gruppen) kann Poly(mercaptopropyl)siloxan im Zuge der Lagerung (auch unter Inertatmosphäre) über eine Kondensationsreaktion vernetzen. Die polymere Verbindung besitzt daher nur eine eingeschränkte Lagerbeständigkeit (ca. 1 Woche). UV vernetzte NR-Latexfilme (vor Alterung und Gamma-Sterilisation) mit 1 und 2phr Vernetzungsmittel weisen eine Reißfestigkeit von 12 MPa - 15 MPa auf.

Um mögliche Nachreaktionen (v.a. Vernetzung) während der Lagerung der polymeren Vernetzungsmittel möglichst gering zu halten wurden in weiteren Syntheseansätzen Disiloxanmonomere eingesetzt. Die polymere Verbindung ist durch die geringere Konzentration an freien Si-OH-Gruppen durch eine wesentlich höhere Lagerbeständigkeit gekennzeichnet und es wird auch über eine Lagerung von 1 Monat (unter Interatmosphäre) keine Änderung in der Viskosität beobachtet. Der Einfluss von unterschiedlichen Parametern (u.a. Reaktionszeit, Monomergehalt) in der Synthese auf die entsprechenden mechanischen Festigkeiten und Alterungsbeständigkeiten von NR-Latexfilmen wurde weiter untersucht.

Im ersten Schritt wurde die Synthese von Poly(mercaptopropylmethyl)siloxan bei einer konstanten Monomerkonzentration in der Reaktionsmischung (9% (w/v)) nach unterschiedlichen Reaktionszeiten (3, 6 und 9 Stunden) abgebrochen, das polymere Produkt aufgearbeitet und entsprechende Vernetzungsexperimente durchgeführt.

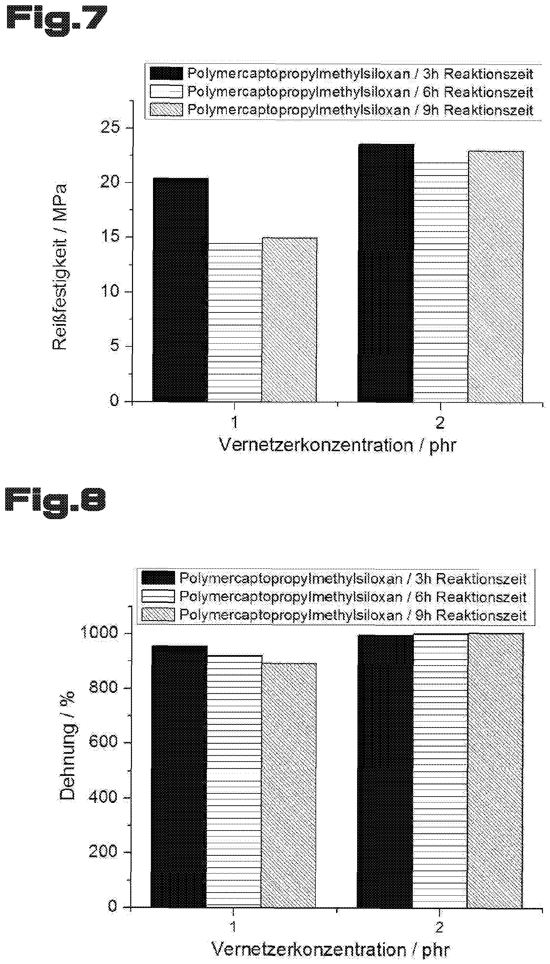

Bei einer Vernetzungsmittelkonzentration von 1 phr, weist das Polymer mit der geringeren Reaktionszeit (3 Stunden) die besseren mechanischen Festigkeiten auf (20 ± 2 MPa). Bei höheren Konzentrationen (2 phr) der polymeren Vernetzungsmittel kann jedoch nur ein geringfügiger Unterschied in den mechanischen Festigkeiten beobachtet werden und die Werte liegen in einem Bereich von 22 bis 24 MPa. Die Ergebnisse sind in den Fig. 7 bis 9 dargestellt.

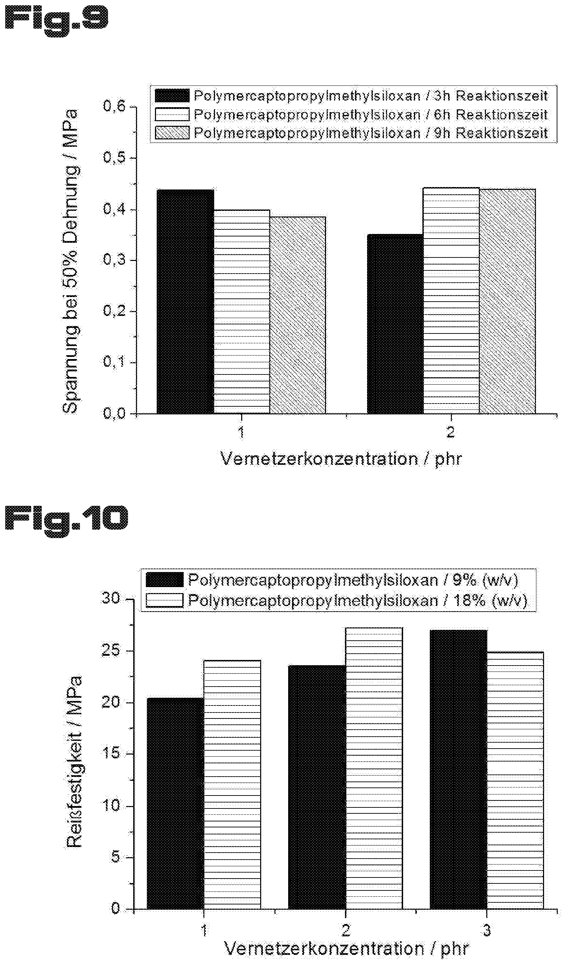

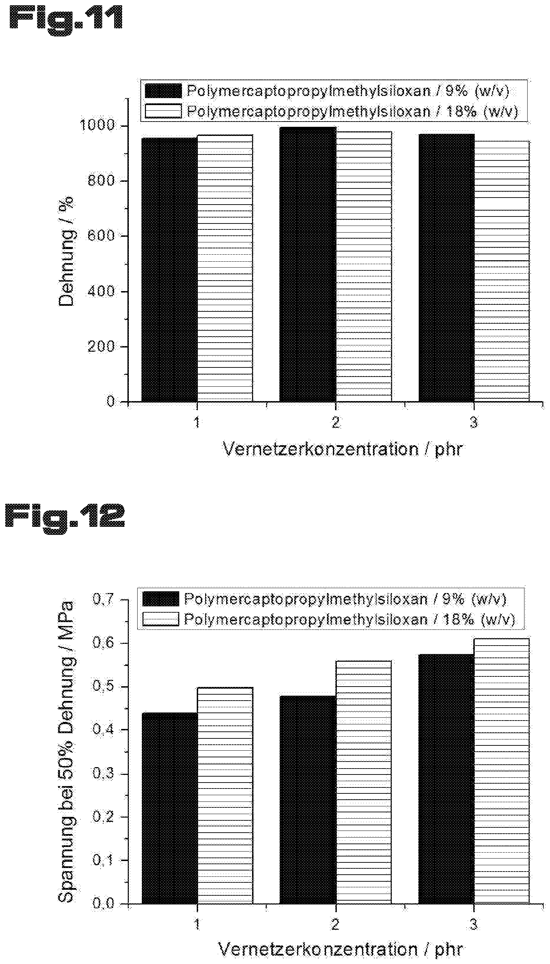

In weiterführenden Arbeiten wurde die Monomerkonzentration in der Synthese bei gleichbleibender Reaktionszeit von 3 Stunden variiert (9 und 18% (w/v)). Während bei geringeren Monomerkonzentrationen (9 (w/v)) eine Zunahme der Reißfestigkeit (von 20 auf 26 ± 2 MPa) mit zunehmender Vernetzerkonzentration in der Latexmischung (von 1 auf 3 phr) beobachtet werden kann, wird bei einer höheren Monomerkonzentration (18% (w/v)) ein Optimum bei 2 phr des Vernetzers in der Latexmischung erzielt (27 ± 2 MPa) erzielt. Die Ergebnisse dieser Untersuchung sind in den Figuren 10 bis 12 dargestellt.

Zusätzlich wurden NR-Latexfilme bei höheren Konzentrationen an polymeren Vernetzungsmitteln hergestellt, UV belichtet und der Einfluss der Vernetzungsmittelkonzentration auf die mechanischen Eigenschaften untersucht. Als polymeres Vernetzungsmittel wurde Poly(mercaptopropylmethyl)siloxan (Monomerkonzentration: 18% (w/v); Reaktionszeit: 3h) gewählt. Die Ergebnisse lassen den Schluss zu, dass eine weitere Erhöhung der Vernetzungsmittelkonzentration von 3 phr auf 4 phr zu keiner signifikanten Verbesserung der Reißfestigkeiten führt. Es kann zwar eine Zunahme des Spannungswertes bei 50% Dehnung erzielt werden, was auf einen höheren Vernetzungsgrad hinweist, aber die Reißfestigkeiten bleiben im Bereich von 25 MPa.

In einem weiteren Schritt wurde ein polymeres Thiolvernetzungsmittel mit kürzerer Zwischengruppe (zwischen Thiolgruppe und polymerer Hauptkette) synthetisiert. Anstelle der Propylgruppe wurde eine Methylgruppe gewählt. Die Monomerkonzentration in der Synthese betrug 9% (w/v) und die Reaktionszeit drei Stunden. Auch diese polymere Verbindung ist lagerstabil und es können keine Viskositätsänderungen über eine Lagerzeit von einem Monat beobachtet werden.

Mit Poly(mercaptomethylmethyl)siloxan als Vernetzungsmittel können im Vergleich zu Poly(mercaptopropylmethyl)siloxan bei gleichen Vernetzerkonzentrationen (1 phr bzw. 2 phr) tendenziell höhere mechanische Festigkeiten bei der UV Vernetzung von NR-Latex erzielt werden. Die Reißfestigkeiten betragen für nicht sterile und nicht gealterte Filme bei einer Konzentration an Poly(mercaptopropylmethyl)siloxan von 1 phr ca. 23 MPa und bei 2 phr ca. 26 MPa. Die 50 % Moduln betragen für nicht sterile und nicht gealterte Filme bei einer Konzentration an Poly(mercaptopropylmethyl)siloxan von 1 phr ca. 0,45 MPa und bei 2 phr ca. 0,5 MPa.

Neben den mercapto-funktionellen Homopolymeren wurden auch Copolymere mit Acryloxypropylmethyleinheiten synthetisiert und als Vernetzungsmittel in der UV Vernetzung von NR-Latex eingesetzt. Durch die Acrylatgruppen als zweite Monomereinheit soll einerseits die Bildung von Disulfiden (als Nebenreaktion der Thiol-En Reaktion) unterbunden werden und andererseits eine reaktive Gruppe (Acrylate) für die Anbindung des polymeren Vernetzungsmittels an die Kautschukkette zur Verfügung stehen. Die Konzentration beider Monomere in der Synthese betrug gesamt 9% (w/v) und die Reaktionszeit drei Stunden. Auch diese polymere Verbindung ist lagerstabil und es können keine Viskositätsänderungen über eine Lagerzeit von einem Monat beobachtet werden. Mit dem Copolymer können im Vergleich zum entsprechenden Homopolymer (Poly(mercaptopropylmethyl)siloxan) bei gleicher Vernetzungsmittelkonzentration in der Latexmischung signifikant höhere Reißfestigkeiten (bis zu 30MPa) erzielt werden.

In weiteren Arbeiten wurde der Einfluss der Comonomerenzusammensetzung auf die mechanischen Eigenschaften untersucht. Hierbei wurde die Konzentration von 3-Acryloxypropylmethylsiloxan von 8,4 auf 16,8 %(mol/total mol) verdoppelt. Die Ergebnisse der Zugprüfung zeigen, dass mit 2 phr P(MPMS-co-APMS) eine Erhöhung der Acrylateinheiten in der Polymerkette mit einer leichten Verringerung von ca. 8% der mechanischen Eigenschaften verbunden ist.

Als Referenz wurde auch ein Acrylathomopolymer synthesiert (analog zu der voranstehend angegeben Synthese der Vernetzungsmittel) und als Vernetzungsmittel eingesetzt. Auf Grund der hohen Reaktivität der Acrylatgruppen wird eine photochemische Vernetzung über direkte C-C-Verknüpfung mit den Isopreneinheiten erzielt. Die erfolgreiche strukturierte Belichtung von Polyisoprenfilmen mit Poly(acryloxypropylmethyl)siloxan als Vernetzungsmittel lässt zwar auf eine ausreichend hohe Reaktivität in der UV-Vernetzung schließen, aber die mechanischen Eigenschaften von entsprechenden NR-Latexfilmen (mit 1 phr und 2 phr Vernetzungsmittel) liegen deutlich niedriger (14 bis 17 MPa) im Vergleich zu den mercaptofunktionellen Homo- und Copolymeren. Zusätzlich sind die Filme durch eine unzureichende Alterungsbeständigkeit (7 Tage Heißluftalterung bei 70°C) gekennzeichnet (<3 MPa und starke Gelbfärbung).

In weiteren Experimenten wurde der Einfluss der Vor- bzw. Nachvernetzung auf die Vernetzung von NR-Latex und IR-Latex mit Poly(mercaptomethylmethyl) siloxan als polymeres Vernetzungsmittel untersucht.

### Vorvernetzung:

Die synthetisierten polymeren Vernetzungsmittel (0,5 phr) wurden mit Lucirin TPO-L (0,5 phr) in deionisiertem Wasser mit Tween 20 (0,1 phr) emulgiert und anschließend dem NR-Latex (40 drc.) bzw. dem IR-Latex (40 drc., Kraton) zugegeben. Die Latexmischung wurde 2 Stunden bei Raumtemperatur gerührt. Anschließend wurde die jeweilige Latexmischung in eine Petrischale gegossen (ca. 1 mm Schichtdicke) und mit einem UV-Strahler von Fusion UV Systems Inc. belichtet. Die NR-Latexmischungen wurden unter Luft mit einem Ga-dotierten Hg-Strahler bei einer Lampenleistung von 60% und einer Bandgeschwindigkeit von 3,5 m/min in vier Durchgängen bestrahlt (entspricht einer Strahlungsdosis von 20,8 J/cm²). Die IR-Latexmischungen wurden unter Luft mit einem Ga-dotierten Hg-Strahler bei einer Lampenleistung von 60% und einer Bandgeschwindigkeit von 3,5 m/min in zwei Durchgängen bestrahlt (entspricht einer Strahlungsdosis von 10,4 J/cm²).

Bei der Herstellung von vorvernetzten Filmen (ohne anschließende Nachvernetzung) wurde die Latexmischung nach der Vorvernetzung mit dem Alterungsschutzmittel (0,5 phr Ionol LC) versetzt und 2 Stunden bei Raumtemperatur gerührt. Anschließend erfolgte die Tauchung der Latexfilme mittels Koagulationstauchverfahren. Hierbei werden nachfolgende Arbeitsschritte durchgeführt:
- Waschen der Keramikformen und Entfetten mit Aceton
- Vorwärmen der Keramikformen für mindestens 10 Minuten im Trockenschrank bei 120 °C
- Tauchen der Form für 30 Sekunden in das Koagulationsbad bei 70 °C
- Trocknen der Form für mindestens 1 Minute im Trockenschrank bei 120 °C
- Tauchen der Form in die NR-Latexmischung für 20 Sekunden
- Trocknen für 20 Minuten im Trockenschrank bei 120 °C
- Abziehen

### Nachvernetzung:

Wahlweise wurde auch eine Nachvernetzung durchgeführt. Hierbei wurden die jeweiligen Latexmischungen (vorvernetzt bzw. nicht vorvernetzt) mit einer Emulsion bestehend aus dem synthetisierten polymeren Vernetzungsmittel (2 phr), Lucirin TPO-L (1 phr), deionisiertem Wasser (2 phr) und Tween 20 (0,1 phr), versetzt. Anschließend wurde die Latexmischung mit einem Alterungsschutzmittel (0,5 phr Ionol LC) versetzt und 2 Stunden bei Raumtemperatur gerührt.

Entsprechende Filme wurden mit Hilfe des Koagulationstauchverfahrens hergestellt und die Nachvernetzung erfolgt im Zuge einer UV-Belichtung der getrockneten Filme (Post-Curing) mit einem UV-Strahler von Fusion UV Systems Inc. Sowohl NR-Latexfilme als auch IR-Latexfilme wurden unter Luft mit einem Ga-dotierten Hg-Strahler bei einer Lampenleistung von 60% und einer Bandgeschwindigkeit von 3,5 m/min in drei Durchgängen bestrahlt (entspricht einer Strahlungsdosis von 15,6 J/cm²).

Bei der photochemischen Vernetzung des NR-Latex zeigte sich, dass die höchsten mechanische Festigkeiten durch eine Nachvernetzung erzielt werden können. Die Reißfestigkeit der NR-Latexfilme (nicht steril, nicht gealtert) betrug ca. 22,5 MPa für die vorvernetzte Probe, ca. 18 MPa für die vor- und nachvernetzte Probe und ca. 25 MPa für die ausschließlich nachvernetzte Probe. Überraschend ist dieses Ergebnis insofern, als die vor- und nachvernetzte Probe die geringste Reißfestigkeit aufwies.

Ein vergleichbarer Trend wird auch bei der UV Vorvernetzung von IR-Latex beobachtet. Hier liegt die Reißfestigkeit von vorvernetzten IR-Latexfilmen im Bereich von 3,5 MPa. Im Gegensatz zu NR-Latexfilmen gelingt aber durch eine kombinierte Vor- und Nachvernetzung eine deutliche Steigerung der Reißfestigkeiten auf 16MPa.

Zur weiteren Evaluierung der polymeren Vernetzungsmittel wurden weitere Mercaptopolymere synthetisiert. Dazu wurde für eine kontrolliertere Polymerisation (Herstellung von Polymeren mit niedrigerem Polydispersitätsindex) die Herstellung von Poly(mercaptomethylmethyl)siloxan und Poly(mercaptopropylmethyl-co-acryloxypropylmethyl)siloxan in Gegenwart von 2 phr bzw. 3 phr Methoxytrimethylsilan als Terminierungsreagenz durchgeführt. Die Synthese erfolgte analog zu voranstehend beschriebener Synthese, wobei zusätzlich 2 phr bzw. 3 phr Methoxytrimethylsilan (Sigma-Aldrich) der Reaktionsmischung zugegeben wurde.

Die Reißfestigkeiten der UV nachvernetzten NR-Latexfilmen (nicht steril, nicht gealtert) liegen durchgehend zwischen 25 MPa und 27 MPa.

Zur Bestimmung der extrahierbaren Vernetzungsmittelkonzentration wurden nachvernetzte NR-Latexfilme im Zuge einer Soxhletextraktion (10 Stunden / Toluol) extrahiert. Das Lösungsmittel wurde mittels Rotavapor abgezogen und der Extrakt im Vakuumtrockenschrank bis zur Gewichtskonstanz bei 35°C und 100 mbar getrocknet.

Mittels C/H/N/S wurde in einer Dreifachbestimmung die extrahierbaren S-Verbindungen (Thiole und Proteine des NR-Latex) bestimmt.

Die Ergebnisse der Elementaranalyse zeigen eine deutlich geringere Extrahierbarkeit (75%) des Vernetzungsmittels im Vergleich zu niedermolekularen Thiolen (wie z.B. Trimethylolpropan-trimercaptopropionat, TMPMP).

**Tabelle 3 - S-Konzentration im Extrakt von UV nachvernetzten NR-Latexfilmen**

| Probe | Photoinitiator | Thiol | Extrahierbarer S-Gehalt / mgS/gLatex |
|---|---|---|---|
| Referenz | Lucirin TPO-L (1phr) | TMPMP (2phr) | 2,074 |
| Homopolymer | Lucirin TPO-L (1phr) | Poly(mercaptopropylmethyl methyl)siloxan (2phr) | 0,534 |
| Copolymer | Lucirin TPO-L (1phr) | Poly(mercaptopropylmethyl-co-acryloxypropylmethyl)siloxan (2phr) | 0,522 |

Die Ausfühungsbeispiele beschreiben mögliche Ausführungsvarianten des Verfahrens.

## Patentansprüche

1. Verfahren zum Herstellen eines Prophylaxeartikels, insbesondere eines Handschuhs, aus einem (carboxylierten) Dienkautschuk, nach dem auf eine Form zumindest eine Schicht aus einem (carboxylierten) Dienlatex aufgebracht wird, und der (carboxylierte) Dienlatex mit einem Vernetzungsmittel vernetzt wird, **dadurch gekennzeichnet, dass** als Vernetzungsmittel ein mercaptofunktionelles Siloxanpolymer verwendet wird.

2. Verfahren nach einem Anspruch 1, **dadurch gekennzeichnet, dass** die Vernetzung der (carboxylierten) Dienlatexmoleküle thermisch und/oder photochemisch mittels ultravioletter Strahlung durchgeführt wird.

3. Verfahren nach einem der Anspruch 1 oder 2, **dadurch gekennzeichnet, dass** der pH-Wert des (carboxylierten) Dienlatex auf einen Wert von größer/gleich 9 eingestellt wird.

4. Verfahren nach einem der Ansprüche 1 bis 3, **dadurch gekennzeichnet, dass** das mercaptofunktionelle Siloxanpolymer dem (carboxylierten) Dienlatex als Emulsion zugesetzt wird.

5. Verfahren nach einem der Ansprüche 1 bis 4, **dadurch gekennzeichnet, dass** als mercaptofunktionelles Siloxanpolymer ein mercaptofunktionelles Siloxanhomopolymer oder ein Copolymer des mercaptofunktionellen Siloxanhomopolymers mit einem Acrylsiloxan verwendet wird.

6. Verfahren nach Anspruch 5, **dadurch gekennzeichnet, dass** ein mercaptofunktionelles Siloxanhomopolymer mit der Strukturformel 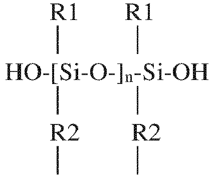 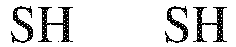 eingesetzt wird, wobei R1 für eine erste Einheit ausgewählt aus einer ersten Gruppe bestehend aus -CH₃, -OH, -C₂H₅, -C₃H₇, aromatische Gruppen, R2 für eine zweite Einheit ausgewählt aus einer zweiten Gruppe bestehend aus -CH₂, C₂H₄, C₃H₆; -(CH₂)₁₁-, aromatische Gruppen, -CH₂-Aromat, steht.

7. Verfahren nach Anspruch 5, **dadurch gekennzeichnet, dass** ein mercaptofunktionelles Siloxancopolymer, insbesondere ein mercaptofunktionelles Siloxancopolymer mit einer statistischen Anordnung der Wiederholungseinheiten, mit der Strukturformel 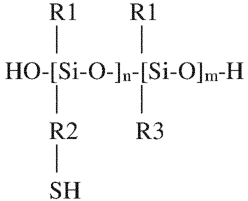 eingesetzt, wobei R1 für eine erste Einheit ausgewählt aus einer ersten Gruppe bestehend aus -CH₃, -OH, -C₂H₅, -C₃H₇, aromatische Gruppen, R2 für eine zweite Einheit ausgewählt aus einer zweiten Gruppe bestehend aus -CH₂, C₂H₄, C₃H₆; -(CH₂)₁₁-, aromatische Gruppen, -CH₂-Aromat, und R3 für eine dritte Einheit ausgewählt aus einer dritten Gruppe bestehend aus Alkylgruppen, -CH₂-Aromat, aromatische Gruppen, Alkengruppen, Methacryloxypropyl-, Acryloxypropyl-, Epoxygruppen steht.

8. Verfahren nach einem der Ansprüche 5 bis 7, **dadurch gekennzeichnet, dass** ein Alkoxyacrylsilan mit der Strukturformel 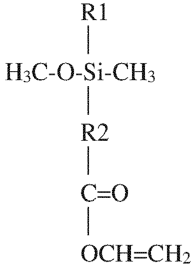 eingesetzt wird, wobei R1 für eine erste Einheit ausgewählt aus einer ersten Gruppe bestehend aus -OH, -CH3, -C₂H₅, -C₃H₇, aromatische Gruppen, R2 für eine zweite Einheit ausgewählt aus einer zweiten Gruppe bestehend aus -CH2, C2H4, C3H6; aromatische Gruppen steht.

9. Verfahren nach einem der Ansprüche 5 bis 7, **dadurch gekennzeichnet, dass** das mercaptofunktionelle Siloxanhomopolymer ausgewählt ist aus einer Gruppe bestehend aus Poly(mercaptopropyl)siloxan, Poly(mercaptopropylmethyl)siloxan, Poly(mercaptomethylmethyl)siloxan, Poly(mercaptoethylmethyl)siloxan, Poly(mercaptomethylethyl)siloxan, Poly(mercaptopropylmethyl)siloxan, Poly(mercaptomethylbenzyl)siloxan, Poly(mercaptopropylbenzyl)siloxan, Poly(mercaptoethylbenzyl)siloxan und/oder dass das Copolymer des mercaptofunktionellen Siloxanhomopolymers mit einem Acrylsiloxan ausgewählt ist aus einer Gruppe bestehend aus Poly(mercaptopropylmethyl-co-acryloxypropylmethyl)siloxan, Poly(mercaptomethylmethyl-co-acryloxymethylpropyl)siloxan, Poly(mercaptomethylmethyl-co-acryloxypropylmethyl)siloxan, Poly(mercaptomethylmethyl-co-acryloxypropylethyl)siloxan, Poly(mercaptomethylmethyl-co-acryloxyethylpropyl)siloxan, Poly(mercaptomethylmethyl-co-acryloxymethylmethyl)siloxan, Poly(mercaptomethylmethyl-co-acryloxypropyl)siloxan, Poly(mercaptomethylmethyl-co-acryloxyethyl)siloxan, Poly(mercaptomethylmethyl-co-acryloxymethyl)siloxan, Poly(mercaptopropylmethyl-co-acryloxymethylpropyl)siloxan.

10. Verfahren nach einem der Ansprüche 5 bis 8, **dadurch gekennzeichnet, dass** der Anteil des mercaptofunktionellen Siloxanpolymers an dem Copolymer des mercaptofunktionellen Siloxanpolymers mit einem Acrylsiloxan zumindest 20 Gew.-% beträgt.

11. Verfahren nach einem der Ansprüche 1 bis 9, **dadurch gekennzeichnet, dass** Vernetzungsmittel dem (carboxylierten) Dienlatex in einem Anteil von 1 phr bis 10 phr, bezogen auf die Gesamtzusammensetzung des (carboxylierten) Dienlatex, zugesetzt wird.

12. Prophylaxeartikel, insbesondere Handschuh, umfassend eine Schicht aus einem (carboxylierten) Dienelastomer, wobei die (carboxylierten) Dienelastomermolekülketten des (carboxylierten) Dienelastomers kovalent über zumindest ein Polymer vernetzt sind, **dadurch gekennzeichnet, dass** das zumindest eine Polymer ein mercaptofunktionelles Siloxanpolymer ist.

## Claims

1. A method for the manufacture of a prophylactic article, in particular of a glove, from a (carboxylated) diene rubber, according to which at least one layer of a (carboxylated) diene latex is applied on a former and the (carboxylated) diene latex is cross-linked with a cross-linking agent, **characterized in that** a mercapto-functional siloxane polymer is used as cross-linking agent.

2. The method according to claim 1, **characterized in that** the cross-linking of the (carboxylated) diene latex molecules is achieved thermally and/or photochemically by means of ultraviolet radiation.

3. The method according to one of claims 1 or 2, **characterized in that** the pH of the (carboxylated) diene latex is adjusted to a value of greater than/equal to 9.

4. The method according to one of claims 1 to 3, **characterized in that** the mercapto-functional siloxane polymer is added as emulsion to the (carboxylated) diene latex.

5. The method according to one of claims 1 to 4, **characterized in that** a mercapto-functional siloxane homopolymer or a copolymer of the mercapto-functional siloxane homopolymer with an acrylic siloxane is used as the mercapto-functional siloxane polymer.

6. The method according to claim 5, **characterized in that** a mercapto-functional siloxane homopolymer with the structural formula 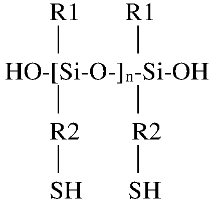 is used, wherein R1 stands for a first unit selected from a first group consisting of -CH₃, -OH, -C₂H₅, -C₃H₇, aromatic groups, R2 for a second unit selected from a second group consisting of -CH₂, C₂H₄, C₃H₆; -(CH₂)₁₁-, aromatic groups, -CH₂-aromatic.

7. The method according to claim 5, **characterized in that** a mercapto-functional siloxane copolymer, in particular a mercapto-functional siloxane copolymer with a statistical arrangement of the repeat units, with the structural formula 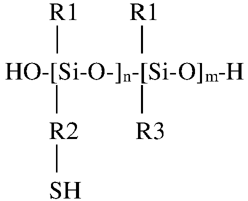 is used, wherein R1 stands for a first unit selected from a first group consisting of -CH₃, -OH, -C₂H₅, -C₃H₇, aromatic groups, R2 for a second unit selected from a second group consisting of -CH₂, C₂H₄, C₃H₆; -(CH₂)₁₁-, aromatic groups, -CH₂-aromatic, and R3 for a third unit, selected from a third group consisting of alkyl groups, -CH₂-aromatic, aromatic groups, alkene groups, methacryloxypropyl-, acryloxypropyl-, epoxy groups.

8. The method according to one of claims 5 through 7, **characterized in that** an alkoxyacrylic silane with the structural formula 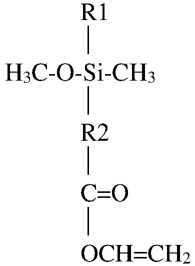 is used, wherein R1 stands for a first unit selected from a first group consisting of -OH, -CH₃, -C₂H₅, -C₃H₇, aromatic groups, R2 for a second unit selected from a second group consisting of -CH₂, C₂H₄, C₃H₆; aromatic groups.

9. Method according to one of claims 5 to 7, **characterized in that** the mercapto-functional siloxane homopolymer is selected from a group consisting of poly(mercaptopropyl) siloxane, poly(mercaptopropylmethyl) siloxane, poly(mercaptomethylmethyl) siloxane, poly(mercaptoethylmethyl) siloxane, poly(mercaptomethylethyl) siloxane, poly(mercaptopropylmethyl) siloxane, poly(mercaptomethylbenzyl) siloxane, poly(mercaptopropylbenzyl) siloxane, poly(mercaptoethylbenzyl) siloxane and/or **in that** the copolymer of the mercapto-functional siloxane homopolymer with an acrylic siloxane selected from a group consisting of poly(mercaptopropylmethyl-co-acryloxypropylmethyl) siloxane, poly(mercaptomethylmethyl-co-acryloxymethylpropyl) siloxane, poly(mercaptomethylmethyl-co-acryloxypropylmethyl) siloxane, poly(mercaptomethylmethyl-co-acryloxypropylethyl) siloxane, poly(mercaptomethylmethyl-co-acryloxyethylpropyl) siloxane, poly(mercaptomethylmethyl-co-acryloxymethylmethyl) siloxane, poly(mercaptomethylmethyl-co-acryloxypropyl) siloxane, poly(mercaptomethylmethyl-co-acryloxyethyl) siloxane, poly(mercaptomethylmethyl-co-acryloxymethyl) siloxane, poly(mercaptopropylmethyl-co-acryloxymethylpropyl) siloxane.

10. The method according to one of claims 5 to 8, **characterized in that** the proportion of mercapto-functional siloxane polymer in the copolymer of the mercapto-functional siloxane polymer with an acrylic siloxane is at least 20 wt%.

11. The method according to one of claims 1 to 9, **characterized in that** the cross-linking agent is added to the (carboxylated) diene latex in a proportion of 1 phr to 10 phr relative to the total composition of the (carboxylated) diene latex.

12. A prophylactic article, in particular a glove, comprising a layer of a (carboxylated) diene elastomer, wherein the (carboxylated) diene elastomer molecular chains of the (carboxylated) diene elastomer are cross-linked covalently via at least one polymer, **characterized in that** the at least one polymer is a mercapto-functional siloxane polymer.

## Revendications

1. Procédé de fabrication d'un article prophylactique, en particulier d'un gant, à partir d'un caoutchouc diène (carboxylé), selon lequel on applique sur une forme au moins une couche d'un latex diène (carboxylé), et on réticule le latex diène (carboxylé) avec un agent de réticulation, **caractérisé en ce qu'**on utilise en tant qu'agent de réticulation un polymère de siloxane mercapto-fonctionnel.

2. Procédé selon la revendication 1, **caractérisé en ce que** la carboxylation des molécules de latex diène (carboxylé) est réalisée par voie thermique et/ou photochimique au moyen d'un rayonnement ultraviolet.

3. Procédé selon la revendication 1 ou 2, **caractérisé en ce que** le pH du latex diène (carboxylé) est ajusté à une valeur supérieure ou égale à 9.

4. Procédé selon l'une des revendications 1 à 3, **caractérisé en ce que** le polymère de siloxane mercapto-fonctionnel du latex diène (carboxylé) est ajouté sous forme d'une émulsion.

5. Procédé selon l'une des revendications 1 à 4, **caractérisé en ce qu'**on utilise en tant que polymère de siloxane mercapto-fonctionnel un homopolymère de siloxane mercapto-fonctionnel ou un copolymère de l'homopolymère de siloxane mercapto-fonctionnel et d'un acrylosiloxane.

6. Procédé selon la revendication 5, **caractérisé en ce qu'**on utilise un homopolymère de siloxane mercapto-fonctionnel de formule développée 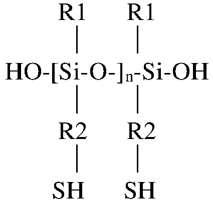 dans laquelle R1 représente un premier motif choisi dans un premier groupe consistant en -CH₃, -OH, -C₂H₅, -C₃H₇, les groupes aromatiques, R2 représente un deuxième motif choisi dans un deuxième groupe consistant en -CH₂, C₂H₄, C₃H₆; -(CH₂)₁₁-, les groupes aromatiques, un groupe -CH₂-aromatique.

7. Procédé selon la revendication 5, **caractérisé en ce qu'**on utilise un copolymère de siloxane mercapto-fonctionnel, en particulier un copolymère de siloxane mercapto-fonctionnel ayant une disposition statistique des motifs répétitifs, de formule développée 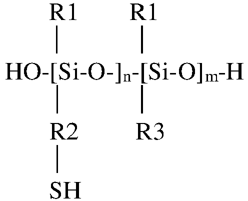 dans laquelle R1 représente un premier motif choisi dans un premier groupe consistant en -CH₃, -OH, -C₂H₅, -C₃H₇, les groupes aromatiques, R2 représente un deuxième motif choisi dans un deuxième groupe consistant en -CH₂, C₂H₄, C₃H₆ ; -(CH₂)₁₁-, les groupes aromatiques, un groupe -CH₂-aromatique, et R3 représente un troisième motif choisi dans un troisième groupe consistant en les groupes alkyle, -CH₂-aromatiques, les groupes aromatiques, les groupes alcène, les groupes méthacryloxypropyle, acryloxypropyle, époxy.

8. Procédé selon l'une des revendications 5 à 7, **caractérisé en ce qu'**on utilise un alcoxyacrylosilane de formule développée 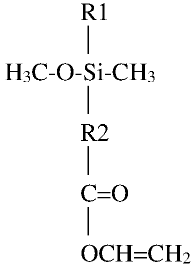 dans laquelle R1 représente un premier motif choisi dans un premier groupe consistant en -OH, -CH₃, -C₂H₅, -C₃H₇, les groupes aromatiques, R2 représente un deuxième motif choisi dans un deuxième groupe consistant en -CH₂, C₂H₄, C₃H₆ ; les groupes aromatiques.

9. Procédé selon l'une des revendications 5 à 7, **caractérisé en ce que** l'homopolymère de siloxane mercapto-fonctionnel est choisi dans un groupe consistant en le poly(mercaptopropyl)siloxane, le poly(mercaptopropylméthyl)siloxane, le poly(mercaptométhylméthyl)siloxane, le poly(mercaptoéthylméthyl)siloxane, le poly(mercaptométhyléthyl)siloxane, le poly(mercaptopropylméthyl)siloxane, le poly(mercaptométhylbenzyl)siloxane, le poly(mercaptopropylbenzyl)siloxane, le poly(mercaptoéthylbenzyl)siloxane et/ou **en ce que** le copolymère de l'homopolymère de siloxane mercapto-fonctionnel et d'un alcoxysiloxane est choisi dans un groupe consistant en le poly(mercaptopropylméthyl-co-acryloxypropylméthyl)siloxane, le poly(mercaptométhylméthyl-co-acryloxyméthylpropyl)siloxane, le poly(mercaptométhylméthyl-co-acryloxypropylméthyl) siloxane, le poly(mercaptométhylméthyl-co-acryloxypropyléthyl)siloxane, le poly(mercaptométhylméthyl-co-acryloxyéthylpropyl)siloxane, le poly(mercaptométhylméthyl-co-acryloxyméthylméthyl)siloxane, le poly(mercaptométhylméthyl-co-acryloxypropyl)siloxane, le poly(mercaptométhylméthyl-co-acryloxyéthyl)siloxane, le poly(mercaptométhylméthyl-co-acryloxyméthyl)siloxane, le poly(mercaptopropylméthyl-co-acryloxyméthylpropyl)siloxane.

10. Procédé selon l'une des revendications 5 à 8, **caractérisé en ce que** la proportion du polymère de siloxane mercapto-fonctionnel, rapportée au copolymère du polymère de siloxane mercapto-fonctionnel et d'un acrylosiloxane, est d'au moins 20 % en poids.

11. Procédé selon l'une des revendications 1 à 9, **caractérisé en ce que** l'agent de réticulation est ajouté au latex diène (carboxylé) selon une proportion de 1 phr à 10 phr, rapportée à la composition totale du latex diène (carboxylé).

12. Article prophylactique, en particulier gant, comprenant une couche d'un élastomère diène (carboxylé), les chaînes de la molécule de l'élastomère diène (carboxylé) de l'élastomère diène (carboxylé) étant réticulées par liaison covalente par l'intermédiaire d'au moins un polymère, **caractérisé en ce que** l'au moins un polymère est un polymère de siloxane mercapto-fonctionnel.
